# EUROPÄISCHE PATENTANMELDUNG

(11) **EP 2 965 740 A1**
(43) Veröffentlichungstag der Anmeldung: **13.01.2016**
(21) Anmeldenummer: 14176730.1
(22) Anmeldetag: 11.07.2014
(51) Int. Cl.: A61K 6/00, A61K 6/083

(54) **Dentalmaterialien mit Debonding-on-Demand-Eigenschaften**

(71) Anmelder: Ivoclar Vivadent AG, 9494 Schaan (LI); Technische Universität Wien, 1040 Wien (AT)
(72) Erfinder: Moszner, Norbert, 9493 Mauren (LI); Rist, Kai, 6800 Feldkirch (AT); Lamparth, Iris, 9472 Grabs (CH); Burtscher, Peter, A-6830 Rankweil (AT); Gorsche, Christian, 1140 Wien (AT); Liska, Robert, 2123 Schleinbach (AT)
(74) Vertreter: Muth, Heinz-Peter

(57) **Zusammenfassung**

Radikalisch polymerisierbarer Dentalwerkstoff, der mindestens eine Verbindung der Formel I: 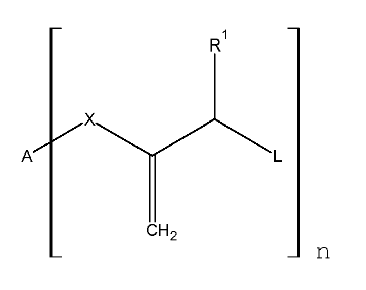 und mindestens eine thermolabile radikalisch polymerisierbaren Verbindung und/oder mindestens eine photolabile radikalisch polymerisierbare Verbindung enthält. Die Werkstoffe zeichnen sich durch Debonding-on-Demand-Eigenschaften aus.

## Beschreibung

Die vorliegende Erfindung betrifft thermisch- und/oder lichthärtende Materialien mit Debonding-on-Demand (DoD) Eigenschaften, die sich insbesondere als dentale Adhäsive oder Zemente beispielsweise zur Befestigung von Inlays, Onlays, Kronen oder Brücken eignen.

Dentale Kompositzemente enthalten eine polymerisierbare organische Matrix und einen oder mehrere Füllstoffe, die meist mit einem polymerisationsfähigen Haftvermittler oberflächenmodifiziert sind. Je nach Art der Füllstoffe, der Monomermatrix und der Anwendung kann der Füllgrad zwischen ca. 20-80 Gew.-% variieren.

Die polymerisierbare organische Matrix enthält in der Regel eine Mischung von Monomeren, Initiator-Komponenten, Stabilisatoren und Pigmenten. Als Harze werden meistens Mischungen von Dimethacrylaten eingesetzt. Beispiele hierfür sind die hochviskosen Dimethacrylate 2,2-Bis[4-(2-hydroxy-3-methacryloyl-oxypropyl)phenyl]propan (Bis-GMA) und 1,6-Bis-[2-methacryl-oyloxyethoxycarbonylamino]-2,4,4-trimethylhexan (UDMA) oder die als Verdünnermonomere genutzten niedrigerviskosen Dimethacrylate, wie z.B. Bismethacryloyloxymethyltricyclo-[5.2.1.]decan (TCDMA), Decandiol-1,10-dimethacrylate (D₃MA) und Triethylenglycoldimethacrylat (TEGDMA).

Bei der radikalischen Polymerisation von dentalen Kompositzementen auf der Basis von vernetzenden Dimethacrylaten kommt es am sogenannten Gelpunkt schon innerhalb von wenigen Sekunden zur Bildung eines 3-dimensionalen polymeren Netzwerkes und damit zur Ausbildung einer hohen mechanischen Festigkeit, so dass ein einfaches, zerstörungsfreies Lösen der mit diesen Zementen verbundenen Substrate oder Teile, wie z.B. die Verbindung eines Zahnstumpfs mit einer Keramikkrone, nur schwer oder gar nicht möglich ist. Die frühe Ausbildung eines 3-dimensionalen Netzwerkes hat zudem eine relativ hohe Polymeri-sationsschrumpfungsspannung (PKS) zur Folge, weil der Polymerisationsschrumpf nicht mehr durch ein viskoses Fliessen der Monomere kompensiert werden kann.

Wiederlösbare Klebeverbindungen haben in verschiedenen Bereichen der Technik eine zunehmende Bedeutung. Beispiele sind das Entfügen von Bauteilen im Rahmen von automatisierten Fertigungsprozessen, die Reparatur von komplexen Bauteilen mit geklebten Teilkomponenten oder die Vereinfachung der stofflichen Trennung beim Recycling solcher Bauteile am Produktlebensende. Das Lösen (debonding) von Klebeverbindungen kann gezielt (on demand) beispielsweise dadurch erreicht werden, dass die Festigkeit der adhäsiven Verbundschicht durch Erwärmung deutlich verringert wird.

So beschreibt die DE 198 32 629 A1 ein Klebstoffsystem zur Bildung reversibler Klebeverbindungen auf Basis von Polyurethanen, Polyharnstoffen oder Epoxidharzen, bei der eine Zusatzkomponente durch Energieeintrag so aktiviert werden kann, dass ein Abbau der Klebstoffkomponenten erfolgt. Beispielsweise können durch Eintrag von Wärme- oder Strahlungsenergie aus blockierten Precursoren organische Basen oder Säuren freigesetzt werden, die einen Abbau des Kleberharzes bewirken.

Die WO 2010/128042 A1 beschreibt technische Klebstoffzusammensetzungen für lösbare Klebeverbindungen im Flugzeug- oder Fahrzeugbau, die aus einer üblichen Klebstoffmatrix und einem partikelförmigen Expansionsstoff wie z.B. Azodicarbonamid bestehen. Dabei erfolgt das Lösen der Bauteile durch Erwärmen der Klebeverbindung mindestens auf die Expansionstemperatur des Expansionsstoffes.

Im Dentalbereich ist das Lösen von Klebeverbindungen unter anderem in der Kieferorthopädie von Bedeutung, wo Brackets, die zur Korrektur von Zahnfehlstellungen auf die Zahnoberfläche geklebt werden, nach erfolgter Korrektur ohne Schädigung des Zahnschmelz wieder entfernen werden müssen. Ausserdem wären im Falle der Reparatur oder des vollkommenen Ersatzes von hochfesten keramischen Restaurationen oder Kronen, die mechanisch nur aufwändig entfernbar sind, einfach erweichbare oder trennbare Zementverbunde von Vorteil.

Im Zusammenhang mit orthodontischen Anwendungen beschreibt die US 2007/0142498 A1 dentale Zusammensetzungen, die thermisch steuerbare Additive wie z.B. thermoplastische Polymere enthalten.

Die US 2007/0142497 A1 offenbart dentale Zusammensetzungen auf Basis von Dimethacrylaten mit säurelabilen tertiären Carbonatgruppen und Photosäuren wie z.B. Triarylsulfoniumsalzen. Diese Zusammensetzungen lassen sich mit geeigneten Initiatoren wie etwa dem Bisacylphosphinoxid Irgacure 819 mit Licht im sichtbaren Bereich photochemisch härten (Photobonding) und durch Bestrahlung mit UV-Licht bei erhöhter Temperatur wieder erweichen (photothermisches Debonding).

Die WO 2013/034777 A2 und die WO 2013/034778 A2 offenbaren Dentalwerkstoffe, die thermolabile oder photolabile polymerisierbare Verbindungen enthalten. Die Werkstoffe lassen sich durch Wärmezufuhr oder durch Bestrahlung mit Licht im UV- oder sichtbaren Wellenlängenbereich wieder vom Substrat lösen.

Der Erfindung liegt die Aufgabe zugrunde, polymerisierbare Dentalwerkstoffe mit Debonding-on-Demand-Eigenschaften bereitzustellen, die eine gute Substrathaftung insbesondere auf Zahnhartsubstanz und/oder Dentalkeramiken zeigen, die sich durch eine verringerte Polymerisationsschrumpfungsspannung (PKS) und eine verbesserte Schlagzähigkeit auszeichnen und die sich vor allem als Adhäsive oder Kompositzemente eignen.

Die Aufgabe wird erfindungsgemäss durch Zusammensetzungen gelöst, die mindestens eine Verbindung der Formel I enthalten, vorzugsweise in Kombination mit einer thermolabilen radikalisch polymerisierbaren Verbindung und/oder einer photolabilen radikalisch polymerisierbaren Verbindung:

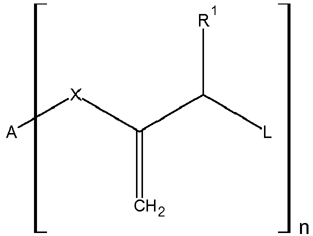

Dabei bedeuten:
- A: H; -CN; ein Phenylrest, der einen oder mehrere Substituenten, wie CH₃, C₂H₅, OH, OCH₃, O-COCH₃, eine polymerisationsfähige Vinyl-, (Meth)acryloyloxy- oder (Meth)acrylamidgruppe tragen kann; oder ein aliphatischer linearer oder verzweigter C₁-C₂₀-Alkylen-Rest, der durch ein oder mehrere 1,4-Phenylengruppen, Urethangruppen, O oder S unterbrochen sein kann und der endständig eine polymerisationsfähige Vinyl-, (Meth)acryloyloxy- oder (Meth)acrylamidgruppe tragen kann;
- R¹: H, ein aliphatischer linearer oder verzweigter C₁-C₉-Alkyl-Rest, Tolyl oder Phenyl;
- L: SR², CO-Phenyl, SO₂R³, PO(R⁴R⁵), PO(OR⁶)(R⁷), PO(OR⁸)(OR⁹) oder Halogen, wobei
- R²⁻⁹: jeweils unabhängig voneinander ein Phenylrest, der einen oder mehrere Substituenten, wie CH₃, C₂H₅, OH, OCH₃, -O-COCH₃, eine polymerisationsfähige Vinyl-, (Meth)acryl-oyloxy-, (Meth)acrylamidgruppe, -C(=CH₂)-COOR¹¹ oder -C (=CH₂) -CO-NR¹²R¹³ tragen kann; oder ein aliphatischer linearer oder verzweigter C₁-C₂₀-Alkylen-Rest sind, der durch O oder S unterbrochen sein kann und der endständig eine polymerisationsfähige Vinyl-, (Meth)acryloyloxy-, (Meth)-acrylamidgruppe, -C (=CH₂)-COOR¹¹ oder -C (=CH₂)-CO-NR¹²R¹³ tragen kann, wobei R¹¹⁻¹³ unabhängig voneinander jeweils ein linearer oder verzweigter C₁₋₆-Rest sind;
- X: -COO-, -CON(R¹⁰)- oder entfällt, wobei die Bindung an A über O bzw. N erfolgt und wobei
- R¹⁰: H; oder ein aliphatischer linearer oder verzweigter C₁-C₂₀-Alkylen-Rest ist, der durch ein oder mehrere O oder S unterbrochen sein kann und der endständig eine polymerisationsfähige Vinyl-, (Meth)acryloyloxy-, (Meth)acrylamidgruppe, -C(=CH₂)-COOR¹¹ oder -C(=CH₂)-CO-NR¹²R¹³ tragen kann, wobei R¹¹⁻¹³ unabhängig voneinander jeweils ein linearer oder verzweigter C₁₋₆-Rest sind;
- n: eine ganze Zahl von 1 bis 6.

Bevorzugte Halogene sind Chlor oder Brom.

Die Formel erstreckt sich nur auf solche Verbindungen, die mit der chemischen Valenzlehre vereinbar sind. Beispielsweise kann n dann, wenn A Wasserstoff ist, nur 1 sein. Der Hinweis, dass ein Rest durch einen oder mehrere Aromaten, Urethangruppen, O, S etc. unterbrochen ist, ist so zu verstehen, dass diese Gruppen in die Kohlenstoffkette des Restes eingeschoben werden. Diese Gruppen sind damit beidseitig durch C-Atome begrenzt und können nicht endständig sein. C₁-Reste können nicht unterbrochen sein.

Die Verbindungen der Formel I enthalten mindestens eine radikalisch polymerisierbare Gruppe, wobei Verbindungen mit 2 bis 8 und insbesondere Verbindungen mit 2 bis 4 radikalisch polymerisierbaren Gruppen bevorzugt sind.

Vorzugsweise haben die Variablen die folgenden Bedeutungen:
- A: H, -CN, ein Phenylrest, ein aliphatischer linearer oder verzweigter C₁-C₁₅-Alkyl-Rest, der durch eine oder mehrere 1,4-Phenylengruppen, Urethangruppen oder O unterbrochen sein kann und der endständig eine polymerisationsfähige (Meth)acryloyloxygruppe tragen kann;
- R¹: H, Phenyl, Tolyl, ein aliphatischer linearer C₁-C₃-Alkyl-Rest;
- L: SR² oder SO₂R³, wobei
- R²⁻³: unabhängig voneinander jeweils ein aliphatischer linearer oder verzweigter C₁-C₂₀-Alkylen-Rest, der durch O unterbrochen sein kann und der endständig eine polymerisationsfähige (Meth)acryloyloxygruppe oder -C(=CH₂)-COOR¹¹ tragen kann, wobei R¹¹ ein linearer oder verzweigter C₁₋₃-Rest ist; oder ein Phenylrest, der einen oder mehrere Substituenten, vorzugsweise CH₃, C₂H₅, OCH₃ und/oder O-COCH₃ tragen kann;
- X: -COO- oder -CON(R¹⁰)-, wobei R¹⁰ Methyl ist, oder entfällt;
- n: 1 oder 2.

Besonders bevorzugt sind Verbindungen der Formel I, in denen mindestens eine und vorzugsweise alle Variablen die folgende Bedeutung haben:
- A: ein gesättigter, linearer aliphatischer Kohlenwasserstoffrest mit 1 bis 12 Kohlenstoffatomen, der durch eine oder mehrere 1,4-Phenylengruppen, Urethangruppen oder O unterbrochen sein und der eine Methacryloyloxygruppe tragen kann,
- X: -COO- oder -CON(R¹⁰)-, wobei R¹⁰ Methyl ist, oder entfällt;
- R¹: H,
- L: -SO₂R³, wobei R³ CH₃ oder Tolyl ist,
- n: 1 oder 2.

Ganz besonders bevorzugt sind Verbindungen der Formel I, in denen die Variablen die folgenden Bedeutungen haben:
- A: ein gesättigter, linearer aliphatischer Kohlenwasserstoffrest mit 6 bis 12 Kohlenstoffatomen, der durch 1 bis 3 O-Atome unterbrochen sein kann,
- X: -COO-;
- R¹: H,
- L: SO₂R³, wobei R³ CH₃ oder Tolyl ist,
- n: 1 oder 2.

Polymerisationsübertragungsaktive Verbindungen der Formel I sind teilweise bekannt und lassen sich nach bekannten Synthesemethoden einfach herstellen. So lassen sich die Iod-Verbindungen I-L wie unten gezeigt an ein ungesättigtes Derivat addieren, und nachfolgend werden durch HI-Abspaltung die erfindungsgemässen Verbindungen der Formel I erhalten:

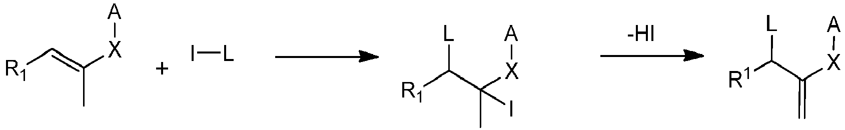

Ein konkretes Beispiel ist:

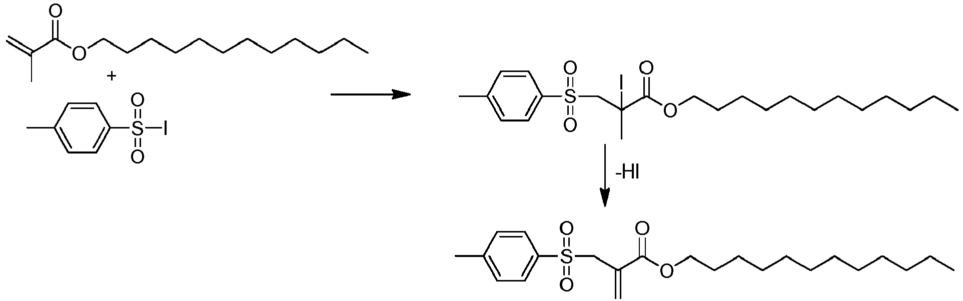

Bevorzugte Beispiele für die erfindungsgemässen polymerisationsübertragungsaktiven Verbindungen der Formel I sind:

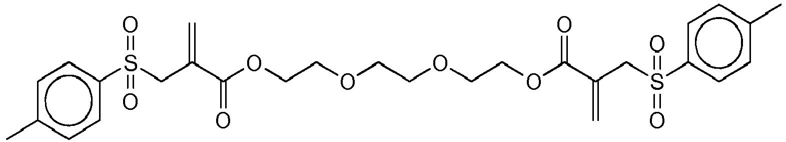

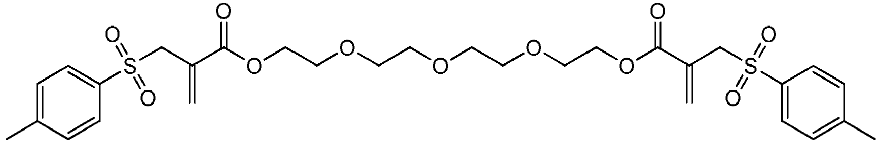

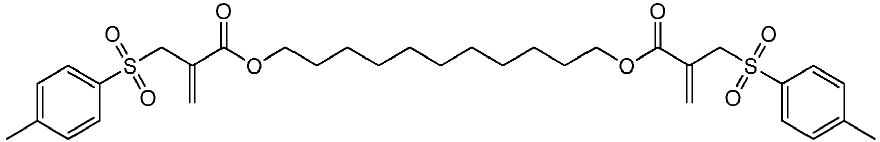

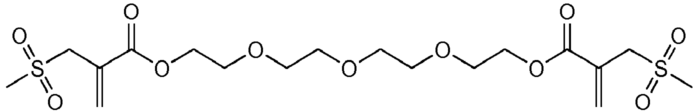

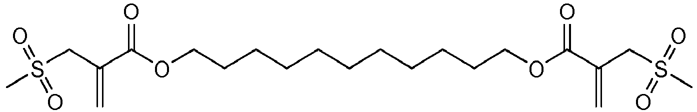

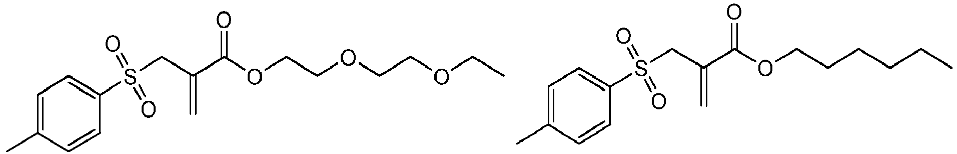

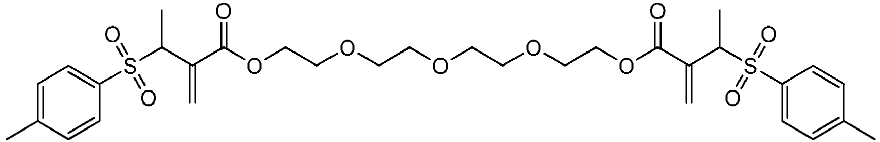

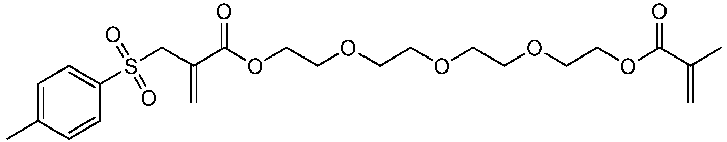

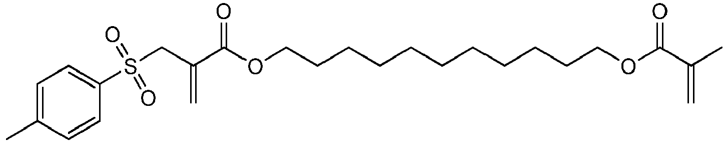

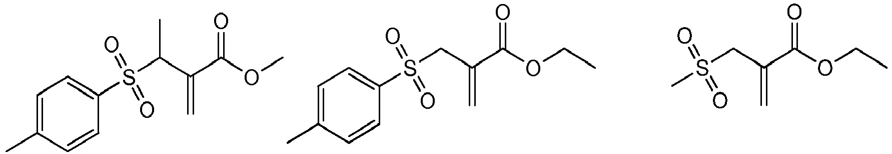

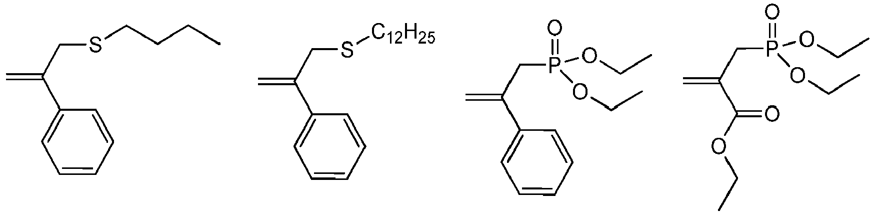

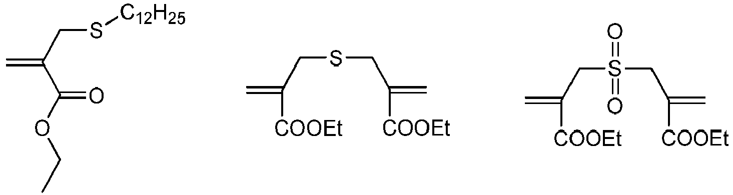

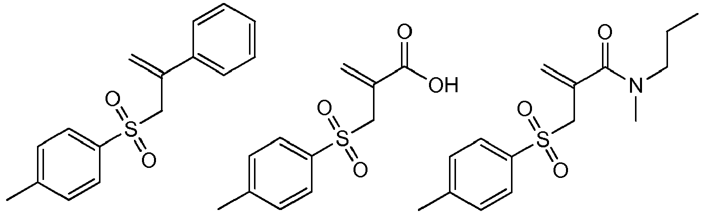

Die Verbindungen der Formel I ermöglichen eine Kontrolle bzw. Steuerung der Netzwerkstruktur bei der radikalischen Polymerisation. Sie führen zu einer deutlich verzögerten Gelbildung und damit zu einer längeren Gelzeit, d.h. dass sich das 3-dimensionale Polymernetzwerk später bildet. Dementsprechend wird eine geringere PKS bei der Aushärtung der Harze bzw. entsprechender Komposite erzielt, was für eine dentale Anwendung z.B. als Füllungsmaterial ein großer Vorteil ist. Die polymerisationsübertragungsaktiven Verbindungen der Formel I ergeben darüber hinaus überraschenderweise auch homogenere Polymernetzwerke mit einem engeren Glasübergang, d.h., dass der Glasübergang in einem engeren Temperaturbereich stattfindet. Dies hat den Vorteil, dass Kettenspannungen durch Relaxationsprozesse besser abgebaut werden und ein schnelleres Debonding-on-Demand (DoD) bewirkt werden kann. Besonders vorteilhaft ist, dass die Glasübergangstemperatur deutlich verringert wird. Die verringerte Glasübergangstemperatur hat zur Folge, dass die Polymeren bei niedrigeren Temperaturen erweicht werden können. Dies gestattet z.B. im Fall von Adhäsiven und Zementen ein gezieltes Lösen der Klebeverbindung (Debonding-on-Demand) auch unter oralen Bedingungen. Die verringerte Glasübergangstemperatur hat den weiteren Vorteil, dass Polymermaterialien mit verbesserter Schlagzähigkeit erhalten werden.

Um den Debonding-on-Demand-Effekt zu verbessern, enthalten die erfindungsgemäßen Zusammensetzungen neben den Verbindungen der Formel I vorzugsweise mindestens eine thermolabile radikalisch polymerisierbare Verbindung und/oder mindestens eine photolabile radikalisch polymerisierbare Verbindung.

Unter thermolabilen bzw. photolabilen Verbindungen, werden solche Monomere verstanden, die thermisch bzw. durch Strahlung spaltbare Gruppe enthalten. Demgegenüber können mit Verbindungen der Formel I Polymere erhalten werden, die sich durch eine Temperaturerhöhung zwar erweichen lassen, sich aber selber nicht spalten. Kombiniert man thermolabile Monomere mit Verbindungen der Formel I, werden Polymere erhalten, die bei relativ niedrigen Temperaturen erweichen und sich zusätzlich thermisch spalten lassen. Kombiniert man Verbindungen der Formel mit photolabilen Monomeren, so ergeben sich Polymere, die bei relativ niedrigen Temperaturen erweichen und sich zusätzlich photochemisch spalten lassen. Schliesslich lassen sich Verbindungen der Formel I mit Mischungen aus einem thermolabilen Monomer und einem photolabilen Monomer kombinieren, wodurch Polymere erhältlich sind, die eine Kombination dieser Merkmale aufweisen. Erfindungsgemäß wurde gefunden, dass sich eine Kombination von Verbindungen der Formel I mit thermo- und/oder photolabilen Monomeren vorteilhaft auf die Debonding-on-Demand-Eigenschaften der gehärteten Materialien auswirkt.

Besonders geeignete thermolabile bzw. photolabile radikalisch polymerisierbare Verbindungen sind thermolabile multifunktionelle (Meth)acrylate und photolabile multifunktionelle (Meth)acrylate, insbesondere thermolabile Di(meth)acrylate und photolabile Di(meth)acrylate, d.h. Di(meth)acrylate mit mindestens einer thermolabilen oder photolabilen Gruppe. Werkstoffe, die thermolabile Comonomere enthalten, sind bevorzugt. Unter polyfunktionellen (Meth)acrylaten werden Verbindungen mit zwei oder mehr, vorzugsweise 2 bis 4 radikalisch polymerisierbaren Gruppen verstanden. Di(meth)acrylate enthalten dementsprechend 2 (Meth)acrylatgruppen.

Weiter bevorzugt sind thermolabile multifunktionelle (Meth)acrylamide und photolabile multifunktionelle (Meth)acrylamide, insbesondere thermolabile Di(meth)acrylamide und photolabile Di(meth)acrylamide.

Geeignete thermolabile Gruppen sind an sich bekannt. Diese zeichnen sich dadurch aus, dass sie eine oder mehrere thermolabile kovalente Bindungen enthalten. Zu bevorzugten thermolabilen Gruppen mit thermolabilen kovalenten Bindungen gehören thermolabile Cycloadditionsaddukte wie Diels-Alder-Addukte, Hetero-Diels-Alder-Addukte sowie thermolabile Alkoxyamin-, Oximester-, Oximurethan- oder Azogruppen. Beispiele für thermolabile Gruppen sind auch beschrieben in R. J. Wojtecki et al., Nature Materials 2011, 10, 14-27.

Besonders bevorzugt sind solche Verbindungen, bei denen die thermolabile Gruppe an zwei polymerisationsfähige (Meth)acrylamidgruppen und insbesondere (Meth)acrylatgruppen gebunden ist.

Bevorzugte Beispiele für thermolabile Vernetzermonomere sind polyfunktionelle (Meth)acrylate oder (Meth)acrylamide mit mindestens einer thermolabilen Gruppe zwischen zwei (Meth)acryl- bzw. (Meth)acrylamidgruppen. Bevorzugte thermolabile Verbindungen sind Diels-Alder-Addukte wie das Diels-Alder-Addukt aus Furfurylmethacrylat und N-(3-(Methacryloyloxy)propyl)-maleinimid, die Umsetzungsprodukte von N-Hydroxy-(meth)acrylamid mit Di- oder Triisocyanaten wie Hexamethylen-1,6-diisocyanat (HDI), 2,2,4- Trimethylhexamethylen-1,6-diisocyanat oder dem HDI-Trimer, sowie Produkte, die durch stöchiometrische Umsetzung von Di- oder Triisocyanaten mit 1-Hydroxymethylacrylsäureestern wie 1-Hydroxymethylacrylsäureethylester oder mit β-Ketoester(meth)acrylaten wie 2-Acetoacetoxyethylmethacrylat erhalten werden.

Gut geeignet sind auch thermolabile Vernetzermonomere, die Gas freisetzen. Beispiele sind die Veresterungsprodukte von Azobis(4-cyanovaleriansäure) mit Hydroxyalkyl(meth)acrylaten wie Hydroxyethyl(meth)acrylat oder Hydroxypropyl(meth)acrylat oder mit N-(Hydroxyalkyl)(meth)acrylamiden wie N-(5-Hydroxypentyl)-methacrylamid oder N-Methyl-N-(2-hydroxyethyl)acrylamid.

Geeignete photolabile Gruppen sind ebenfalls an sich bekannt. Diese zeichnen sich dadurch aus, dass sie eine oder mehrere photolabile kovalente Bindungen enthalten. Zu bevorzugten photolabilen Gruppen mit photolabilen kovalenten Bindungen gehören Benzoinether, Oxyalkylphenylacetophenone, Dialkyloxyacetophenone, Benzoyldiphenylphosphinoxide, Dibezoylphenylphosphinoxide, Dialkylbenzoyl- und Dialkyldibenzoylgermanium-Derivate. Insbesondere sind solche Verbindungen bevorzugt, die eine photolabile Gruppe und zwei polymerisationsfähige (Meth)acrylatgruppen enthalten. Bevorzugte Verbindungen sind Bis-(4-meth-ocryloyloxybenzoyl-diethylgermanium, Bis-{4-[2-(methacryloyloxy)-diethylcarbamoyloxybenzoyl}diethylgermanium, Bis-[3-(methacryloyloxymethyl)-2,4,6-trimethylbenzoyl]phenylphosphin-oxid und Methacrylsäure-2-[2-(4-{2-methyl-2-[2-(methacryl-oyloxy)-ethylcarbamoyloxy]-propionyl}-phenoxy)-ethoxycarbonyl-amino]-ethylester.

Desweiteren sind die in der WO 2013/034777 A2 und der WO 2013/034778 A2 beschriebenen thermolabilen oder photolabilen Monomere gut geeignet.

Die erfindungsgemäßen Dentalwerkstoffe enthalten, ggf. neben den thermolabilen oder photolabilen polymerisierbaren Verbindungen, vorzugsweise auch ein oder mehrere weitere radikalisch polymerisierbare Monomere, besonders bevorzugt mindestens ein multifunktionelles (Meth)acrylat oder eine Mischungen von mono- und multifunktionellen (Meth)acrylaten. Unter monofunktionellen (Meth)acrylaten werden auch hier Verbindungen mit einer, unter polyfunktionellen (Meth)acrylaten Verbindungen mit zwei oder mehr, vorzugsweise 2 bis 4 radikalisch polymerisierbaren Gruppen verstanden. Gemäß einer ganz besonders bevorzugten Ausführungsform enthalten die erfindungsgemäßen Zusammensetzungen mindestens ein Dimethacrylat oder eine Mischung von Mono- und Dimethacrylaten.

Beispiele für besonders geeignete mono- oder multifunktionelle (Meth)acrylate sind Methyl-, Ethyl-, 2-Hydroxyethyl-, Butyl-, Benzyl-, Tetrahydrofurfuryl- oder Isobornyl(meth)acrylat, p-Cumyl-phenoxyethylenglycolmethacrylat (CMP-1E), Bisphenol-A-di(meth)acrylat, Bis-G(M)A (ein Additionsprodukt aus (Meth)acrylsäure und Bisphenol-A-diglycidylether), ethoxyoder propoxyliertes Bisphenol-A-di(meth)acrylat, wie z.B. das Bisphenol-A-dimethacrylat SR-348c (Sartomer) mit 3 Ethoxygruppen oder 2,2-Bis[4-(2-(meth)acryloxypropoxy)phenyl]propan, UD(M)A (ein Additionsprodukt aus 2-Hydroxyethyl(meth)acrylat und 2,2,4-Trimethylhexamethylendiisocyanat), Di-, Tri- oder Tetraethylenglycoldi(meth)acrylat, Trimethylolpropantri(meth)-acrylat, Pentaerythrittetra(meth)acrylat, sowie Glycerindi-und tri(meth)acrylat, 1,4-Butandioldi(meth)acrylat, 1,10-Decandioldi(meth)acrylat (D₃MA) oder 1,12-Dodecandioldi-(meth)acrylat.

Die erfindungsgemässen dentalen Werkstoffe können neben den oben genannten Co-Monomeren vorzugweise auch radikalisch polymerisierbare, säuregruppenhaltige Monomere (Haftmonomere) enthalten. Bevorzugte Säuregruppen sind Carbonsäuregruppen, Phosphonsäuregruppen, Phosphorsäuregruppen und Sulfonsäuregruppen. Bevorzugte Monomere mit Carbonsäuregruppen sind Maleinsäure, Acrylsäure, Methacrylsäure, 2-(Hydroxymethylacrylsäure, 4-(Meth)acryloyloxyethyltrimellitsäureanhydrid, 10-Methacryloyloxydecylmalonsäure, N-(2-Hydroxy-3-methacryloyloxypropyl)-N-phenylglycin und 4-Vinylbenzoesäure. Bevorzugte Monomere mit Phosphonsäuregruppen sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentylphosphonsäure, 2-[4-(Dihydroxyphosphoryl)-2-oxa-butyl]-acrylsäure, 2-[4-(Dihydroxyphosphoryl)-2-oxa-butyl]-acrylsäureethyl- und -2,4,6-trimethylphenylester. Bevorzugte Monomere mit Phosphorsäuregruppen sind 2-Methacryloyloxypropylmono- oder -dihydrogenphosphat, 2-Methacryloyloxyethylmono- oder -dihydrogenphosphat, 2-Methacryloyloxyethylphenylhydrogenphosphat, Dipentaerythritolpentamethacryloyloxyphosphat, 10-Methacryloyloxydecyl-dihydrogenphosphat, Phosphorsäuremono-(1-acryloyl-piperidin-4-yl)-ester, 6-(Methacrylamidohexyldihydrogenphosphat und 1,3-Bis-(N-acryloyl-N-propylamino)-propan-2-yl-dihydrogenphosphat. Bevorzugte Monomere mit Sulfonsäuregruppen sind Vinylsulfonsäure, 4-Vinylphenylsulfonsäure und 3-(Methacrylamido)propylsulfonsäure.

Zur Initiierung der radikalischen Polymerisation enthalten die erfindungsgemäßen Zusammensetzungen vorzugsweise einen Initiator für die radikalische Polymerisation, besonders bevorzugt einen Photoinitiator. Als Photoinitiatoren eignen sich insbesondere Benzophenon, Benzoin sowie deren Derivate oder α-Diketone oder deren Derivate, wie 9,10-Phenanthrenchinon, 1-Phenyl-propan-1,2-dion, Diacetyl oder 4,4'-Dichlorbenzil. Bevorzugt werden Campherchinon (CQ) und 2,2-Dimethoxy-2-phenyl-acetophenon und besonders bevorzugt α-Diketone in Kombination mit Aminen als Reduktionsmittel, wie z.B. 4-(Dimethylamino)-benzoesäureester (EDMAB), N,N-Dimethylaminoethylmethacrylat, N,N-Dimethyl-sym.-xylidin oder Triethanolamin, verwendet. Geeignet sind auch Norrish-Typ-I-Photoinitiatoren, vor allem Acyl- der Bisacylphosphinoxide, und besonders geeignet Monoacyltrialkyl- bzw. Diacyldialkylgermanium-Verbindungen, wie z.B. Benzoyltrimethylgermanium, Dibenzoyldiethylgermanium oder Bis(4-methoxybenzoyl)diethylgermanium (MBDEGe). Dabei lassen sich auch Mischungen der verschiedenen Photoinitiatoren einsetzen, wie z.B. Bis(4-methoxybenzoyl)diethylgermanium in Kombination mit Campherchinon und 4-Dimethylaminobenzoesäureethylester.

Gemäß einer bevorzugten Ausführungsform enthalten die erfindungsgemäßen Dentalwerkstoffe zusätzlich organischen oder anorganischen partikulären Füllstoff, besonders bevorzugt einen oder mehrere anorganische partikuläre Füllstoffe.

Besonders geeignet sind Füllstoffe auf der Basis von Oxiden mit einer Partikelgröße von 0,01 bis 15 µm, wie SiO₂, ZrO₂ und TiO₂ bzw. Mischoxiden aus SiO₂, ZrO₂, ZnO und/oder TiO₂, nanopartikuläre oder mikrofeine Füllstoffe mit einer Partikelgröße von 10 bis 300 nm, wie pyrogene Kieselsäure oder Fällungskieselsäure sowie Glaspulver mit einer Partikelgröße von 0,01 bis 15 µm, vorzugweise von 0,2 bis 1,5 µm, wie Quarz-, Glaskeramikoder röntgenopake Glaspulver von z.B. Barium- oder Strontiumaluminiumsilikatgläsern, und röntgenopake Füllstoffe mit einer Partikelgröße von 0,2 bis 5 µm, wie Ytterbiumtrifluorid, Tantal(V)-oxid, Bariumsulfat oder Mischoxide von SiO₂ mit Ytterbium(III)-oxid oder Tantal(V)-oxid. Auch faserförmige Füllstoffe, Nanofasern oder Whiskers sind nicht ausgeschlossen. Wenn nicht anders angegeben handelt es sich bei allen Partikelgrößen um gewichtsmittlere Partikelgrößen.

Füllstoffe werden nach der Partikelgröße unterteilt in Makrofüller und Mikrofüller. Makrofüller werden durch Mahlen von Quarz, röntgenopaken Gläsern, Borosilikaten oder von Keramik gewonnen, sind rein anorganischer Natur und bestehen meist aus splitterförmigen Teilen. Bevorzugt sind Makrofüller mit einer mittleren Partikelgröße von 0,2 bis 10 µm. Als Mikrofüller werden vorzugsweise pyrogenes SiO₂ oder Fällungskieselsäure eingesetzt, oder auch Mischoxide, z.B. SiO₂-ZrO₂, die durch hydrolytische Cokondensation von Metallalkoxiden zugänglich sind. Die Mikrofüller haben vorzugsweise eine mittlere Partikelgröße von ca. 5 bis 100 nm.

Der bevorzugte Füllstoffgehalt richtet sich dabei nach der gewünschten Anwendung. Adhäsive enthalten vorzugsweise 0 bis 20 Gew.-% und Zemente und Komposite vorzugsweise 20 bis 80 Gew.-% Füllstoff, jeweils bezogen auf die Gesamtmasse des Werkstoffs.

Zur Verbesserung des Verbundes zwischen den Füllstoffpartikeln und der vernetzten Polymerisationsmatrix können SiO₂-basierende Füllstoffe mit (Meth)acrylat-funktionalisierten Silanen oberflächenmodifiziert werden. Ein bevorzugtes Beispiel für solche Silane ist 3-(Meth)acryloyloxypropyltrimethoxysilan. Zur Oberflächenmodifizierung von nichtsilikatischen Füllstoffen wie z.B. von ZrO₂ oder TiO₂ können auch funktionalisierte saure Phosphate, wie z.B. 10-(Meth)acryloyloxydecyldihydrogenphosphat eingesetzt werden.

Die erfindungsgemässen Dentalwerkstoffe können zudem ein thermisch gasfreisetzendes Additiv enthalten. Bevorzugte gasfreisetzende Additive sind z.B. Azo-Verbindungen wie Azodicarbonamid, 2,2'-Azobisisobutyronitril oder 2,2'-Azobis(4-cyano-pentansäure), N-Nitrosoverbindungen, Hydrazide wie Benzolsufonylhydrazid, Peroxide wie Dicumolperoxid oder Acetondicarbonsäure. Beispiele für solche Verbindungen sind etwa in St. Quinn, Plastics, Additives & Compounding 2001, 3, 16-21 beschrieben. Dabei kann die Zerfallstemperatur beispielsweise im Falle der Azoverbindungen in an sich bekannter Weise durch das Substituentenmuster eingestellt werden (vgl. D. Braun, R. Jakobi, Monatshefte Chemie 1982, 113, 1403-1414).

Weiterhin können die erfindungsgemässen Dentalwerkstoffe ein Additiv enthalten, das eingestrahlte elektromagnetische Strahlung in Wärme umwandeln kann. Solche sogenannten Strahlung-Wärme-Umwandler sind organische, anorganische oder metallorganische Stoffe oder Hybridkomponenten, die in der Lage sind, UV-, NIR- oder IR-Strahlung, sichtbares Licht, Mikrowellenoder Radiowellenstrahlung in Wärme umzuwandeln, um thermolabile Gruppen zu spalten. Beispiele hierfür sind UV-, NIR oder IR-Strahlung absorbierende Farbstoffe und Pigmente. Beispiele für im IR-Bereich absorbierende Farbstoffe sind Azo-, Methin-, Anthrachinon oder Porphyrinfarbstoffe. Beispiele für NIR-Strahlung absorbierende Pigmente sind Antimon- und Indiumzinnoxid, Phthalocyaninpigmente, Ruß, Ni- und Pt-Dithiolen-Komplexe. Beispiele für im UV-Bereich absorbierende Verbindungen sind Benzotriazole, Triazine, Benzophenone, Cyanoacrylate, Salicylsäurederivate und Hindered Amine Light Stabilizers (HALS). Beispiele für Additive, die im Frequenzbereich der Mikrowellen (1 bis 300 GHz) oder Radiowellen (10 kHz bis 1 GHz) absorbieren, sind ferromagnetische keramische Stoffe, sogenannte Ferrite, die aus den Eisenoxiden Hämatit (Fe₂O₃) oder Magnetit (Fe₃O₄) und weiteren Oxiden beispielweise der Metalle Zn, Mn, oder Ni aufgebaut und als Pulver kommerziell verfügbar sind.

Gegebenenfalls können die erfindungsgemäß eingesetzten Zusammensetzungen ein oder mehrere weitere Additive enthalten, vor allem Lösungsmittel wie Wasser, Ethanol oder entsprechende Lösungsmittelgemische, Stabilisatoren, Farbmittel, fluoridionenabgebende Additive, optische Aufheller, Weichmacher oder UV-Absorber.

Der bevorzugte Lösungsmittelgehalt richtet sich dabei nach der gewünschten Anwendung. Adhäsive enthalten vorzugweise bevorzugt 0 bis 60 Gew.-% und besonders bevorzugt 1 bis 50 Gew.-% Lösungsmittel. Demgegenüber enthalten Zemente vorzugsweise kein Lösungsmittel.

Die Debonding-Eigenschaften der erfindungsgemässen Dentalwerkstoffe können durch die Zusammensetzung der Materialien gezielt beeinflusst und an den gewünschte Anwendungszweck angepasst werden. So nimmt mit der verwendeten Konzentration an thermolabilen oder photolabilen Komponenten, d.h. insbesondere der thermolabilen oder photolabilen Vernetzermonomere und gasfreisetzenden Additive, die Fähigkeit zum gezielten Debonding durch Erwärmen oder Bestrahlen zu. Weiterhin lassen sich die Debonding-Eigenschaften auch durch die Auswahl der Co-Monomere variieren, wobei mit dem Anteil an vernetzenden Monomeren oder durch Zugabe von monofunktionellen Monomeren die Vernetzungsdichte und damit auch die Festigkeit und der E-Modul variiert werden können.

Die erfindungsgemäßen Dentalmaterialien enthalten vorzugsweise 0,5 bis 60 Gew.-%, bevorzugt 1,0 bis 50 Gew.-% und besonders bevorzugt 1,0 bis 40 Gew.-% mindestens einer Verbindung der allgemeinen Formel I.

Zusätzlich enthalten die Materialien vorzugsweise auch 0,01 bis 5,0 Gew.-%, bevorzugt 0,1 bis 5,0 Gew.-% und besonders bevorzugt 0,1 bis 3,0 Gew.-% Initiator(en) für die radikalische Polymerisation, besonders bevorzugt einen Photoinitiator, und besonders bevorzugt auch 1 bis 60 Gew.-%, bevorzugt 5 bis 50 Gew.-% und besonders bevorzugt 5 bis 40 Gew.-% difunktionelle thermo- und/oder photolabile (Meth)acrylat(e).

Weiterhin enthalten die erfindungsgemäßen Dentalwerkstoffe vorzugsweise 0 bis 80 Gew.-%, bevorzugt 0 bis 70 Gew.-% und besonders bevorzugt 0 bis 60 Gew.-% Füllstoff(e), wobei der Füllstoffgehalt wie oben beschrieben auf die geplante Verwendung der Werkstoffe abgestimmt wird.

Außerdem enthalten die erfindungsgemäßen Dentalwerkstoffe vorzugsweise 0 bis 40 Gew.-%, bevorzugt 0 bis 30 Gew.-% und besonders bevorzugt 5 bis 30 Gew.-% expandierende Additiv(e) und/oder Strahlungs-Wärmeumwandler, und ggf. 0 bis 5 Gew.-%, bevorzugt 0 bis 3 Gew.-%, besonders bevorzugt 0,5 bis 3 Gew.-% sonstige Additiv(e).

Dabei sind erfindungsgemäß Dentalmaterialien besonders bevorzugt, welche die folgenden Komponenten enthalten:
(a) 1 bis 80 Gew.-%, bevorzugt 10 bis 70 Gew.-% und besonders bevorzugt 10 bis 60 Gew.-% multifunktionelle (Meth)-acrylat(e),
(b) 0,01 bis 5,0 Gew.-%, bevorzugt 0,1 bis 5,0 Gew.-% und besonders bevorzugt 0,1 bis 3,0 Gew.-% Initiator(en),
(c) 0,5 bis 60 Gew.-%, bevorzugt 1,0 bis 50 Gew.-% und besonders bevorzugt 1,0 bis 40 Gew.-% mindestens einer Verbindung der allgemeinen Formel I,
(d) 0 bis 50 Gew.-%, bevorzugt 0 bis 40 Gew.-% und besonders bevorzugt 0 bis 30 Gew.-% monofunktionelle (Meth)-acrylat(e),
(e) 1 bis 60 Gew.-%, bevorzugt 5 bis 50 Gew.-% und besonders bevorzugt 5 bis 40 Gew.-% difunktionelle thermo- und/oder photolabile (Meth)acrylat(e),
(f) 0 bis 80 Gew.-%, bevorzugt 0 bis 70 Gew.-% und besonders bevorzugt 0 bis 60 Gew.-% Füllstoff(e),
(g) 0 bis 40 Gew.-%, bevorzugt 0 bis 30 Gew.-% und besonders bevorzugt 5 bis 30 Gew.-% expandierende Additiv(e) und/oder Strahlungs-Wärmeumwandler,
(h) 0 bis 60 Gew.-%, bevorzugt 0 bis 50 Gew.-%, besonders bevorzugt 1 bis 50 Gew.-% Lösungsmittel und
(i) 0 bis 5 Gew.-%, bevorzugt 0 bis 3 Gew.-%, besonders bevorzugt 0,5 bis 3 Gew.-% sonstige Additiv(e).

Alle Mengenangaben beziehen sich wenn nicht anders angegeben auf die Gesamtmasse der Zusammensetzung. Die einzelnen Mengenbereiche können separat gewählt werden.

Ganz besonders bevorzugt sind solche Dentalwerkstoffe, welche aus den genannten Komponenten bestehen. Weiterhin sind solche Werkstoffe bevorzugt, bei denen die einzelnen Komponenten jeweils aus den oben genannten bevorzugten und besonders bevorzugten Stoffen ausgewählt sind. Besonders bevorzugt sind Werkstoffe, die ggf. neben der Verbindung der Formel (I) keine flüchtigen Mercaptane enthalten, d.h. Mercaptane, die einen typischen Mercaptangeruch aufweisen. Ganz besonders bevorzugt sind Zusammensetzungen, die keine weiteren Mercaptane und vorzugsweise auch keine anderen Disulfide oder Thioether enthalten.

Die erfindungsgemäßen Werkstoffe eignen sich besonders als dentale Adhäsive und Zemente. Sie haben ähnliche mechanische Eigenschaften (Biegefestigkeit und E-Modul) wie auf Dimethacrylaten basierende Materialien, zeichnen sich aber durch eine verringerte Polymerisationsschrumpfungsspannung (PKS), Debonding-on-Demand Eigenschaften, einen engen Glasübergangsbereich, verbesserte Schlagzähigkeit und geringen Eigengeruch aus.

Die Dentalwerkstoffe eignen sich primär zur intraoralen Anwendung durch den Zahnarzt zur Restauration geschädigter Zähne (klinische Materialien). Sie können aber auch extraoral eingesetzt werden, beispielsweise bei der Herstellung oder Reparatur von Dentalrestaurationen (technische Materialien).

Gegenstand der Erfindung ist auch die Verwendung von Verbindungen der Formel I zur Herstellung eines Adhäsivs oder Zements mit Debonding-on-Demand-Eigenschaften.

Die Erfindung wird im Folgenden anhand von Ausführungsbeispielen näher erläutert.

### Beispiel 1:

### Synthese von 2-(Toluol-4-sulfonylmethyl)-acrylsäurelauryl-ester (1)

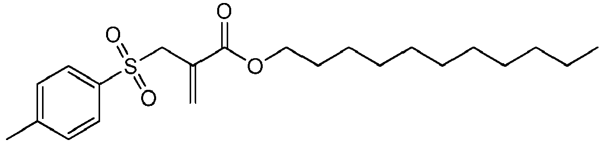

Zunächst wurden im Gelblichtlabor 3,81 g (15 mmol) Iod in 70 ml Ethanol gelöst und langsam in eine 0,1M Lösung von Natrium-p-toluolsulfinat (15 mmol) in Wasser getropft. Der dabei entstehende gelbe Feststoff (4-Methylbenzen-1-sulfonyliodid, MBSI) wurde filtriert und mit Wasser nachgewaschen. Anschließend wurde der Feststoff in CH₂Cl₂ (50 ml) aufgelöst und mit wasserfreiem Na₂SO₄ getrocknet. Das Trockenmittel wurde filtriert, und die Lösung mit dem frisch hergestellten MBSI wurde zusammen mit 2,54 g (10 mmol) Laurylmethacrylat (LMA) gerührt. Die Reaktion wurde mittels Dünnschichtchromatographie (PE/EE 20/1) verfolgt. Nachdem das gesamte LMA aufgebraucht war, wurde die Reaktionslösung mit 5 Gew.-% Natriumdithionit-Lösung (2 x 25 ml) und mit Wasser (1 x 25 ml) gewaschen. Die wässrige Phase wurde mit CH₂Cl₂ rückextrahiert (1 x 25 ml) und die vereinigten organischen Phasen über wasserfreiem Na₂SO₄ getrocknet. Im Anschluss wurden 50 ml Ethylacetat zur Lösung zugegeben und das CH₂Cl₂ wurde am Rotationsverdampfer verdampft. Zur Reaktionslösung wurden nun unter Ar-Atmosphäre 5,06 g (50 mmol) Triethylamin zugegeben und dann über Nacht am Rückfluss gekocht. Nach vollständiger Reaktion wurde die Lösung mit 1N HCl (2 x 50 ml) und dest. Wasser (1 x 50 ml) gewaschen. Die wässrigen Phasen wurden rückextrahiert und die vereinigten organischen Phasen über wasserfreiem Na₂SO₄ getrocknet. Das Lösungsmittel wurde am Rotationsverdampfer abgezogen und das Rohprodukt mit einem Gemisch aus PE/EE 5/1 säulenchromatographisch gereinigt (R_{f} = 0,39). Die Ausbeute betrug ca. 28,8 g (73% der Theorie).
¹H-NMR (200 MHz, CDCl₃, δ): 7.71 (d, *J =* 8.2 Hz, 2H; Ar-H), 7.30 (d, *J* = 8.2 Hz, 2H; Ar-H), 6.47 (s, 1H; =CH₂), 5.89 (s, 1H; =CH₂), 4.12 (s, 2H; -SO₂-CH₂-), 3.94 (t, 2H; -O-CH₂-CH₂-), 2.42 (s, 3H; Ar-CH₃), 1.52 (m, 2H; -O-CH₂-CH₂-), 1.25 (s, 18H; -O-CH₂-CH₂-CH₂-CH₂-CH₂-CH₂-CH₂-CH₂-CH₂-CH₂-CH₂-CH₃); 0.86 (m, 3H; -CH₂-CH₃);
¹³C-NMR (50 MHz, CDCl₃, δ): 164.9 (C=O), 144.8 (C4), 135.5 (C4), 133.1 (C2), 129.6 (C3), 129.2 (C4), 128.8 (C3), 65.6 (C2), 57.5 (C2), 31.9 (C2), 29.6 (C2, C2, C2), 29.5 (C2), 29.3 (C2), 29.2 (C2), 28.4 (C2), 25.8 (C2), 22.7 (C2), 21.6 (C1), 14.1 (C1).

### Beispiel 2:

### Synthese von Tetraethylenglykol-bis[2-(toluol-4-sulfonylmethyl)acrylat] (2)

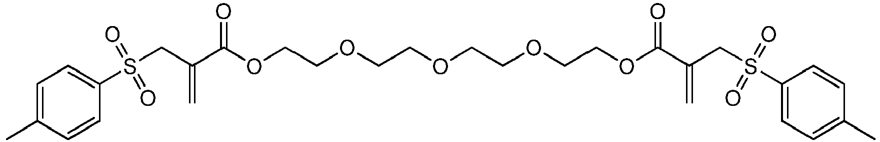

Tetraethylenglycoldimethacrylat (TTEGDMA: 5,29 g, 16 mmol) und 4-Toluolsulfonyliodid (9,03 g, 32 mmol) wurden gemeinsam in CH₂Cl₂ (ca. 50 ml) bei Raumtemperatur im Gelblicht gerührt. Die Reaktion wurde mittels ¹H-NMR-Spektoskopie verfolgt. Nachdem das gesamte TTEGDMA aufgebraucht war (Abnahme der Doppelbindungssignale) wurde die Reaktionslösung mit 5 Gew.-% Natriumdithionit-Lösung (2 x 25ml) und mit Wasser (1 x 25ml) gewaschen. Die wässrige Phase wurde mit CH₂Cl₂ rückextrahiert (1 x 25ml) und die vereinigten organischen Phasen über Na₂SO₄ getrocknet. Im Anschluss wurden 50 ml Ethylacetat zur Lösung zugegeben und das CH₂Cl₂ am Rotationsverdampfer verdampft. Zur Reaktionslösung wurden dann weitere 50 ml Ethylacetat zugegeben und unter Ar-Atmosphäre Triethylamin (8,1 g, 80 mmol) zugetropft (Feststoff fiel aus). Danach wurde die Reaktionslösung über Nacht auf Rückfluss gekocht. Nach vollständiger Reaktion wurde die Lösung mit 1N HCl (2 x 50 ml) und dest. Wasser (1 x 50 ml) gewaschen. Die wässrigen Phasen wurden rückextrahiert und die vereinigten organischen Phasen über Na₂SO₄ getrocknet. Das Lösungsmittel wurde am Rotationsverdampfer abgezogen und das Rohprodukt mit einem Gemisch aus PE/EE 1/4 säulenchromatographisch gereinigt. (R_{f} ∼ 0,32). Ausbeute 70%.
¹H-NMR (200 MHz, CDCl₃, δ): 7.73 (d, *J =* 8.2 Hz, 4H; Ar-H), 7.32 (d, *J* = 8.2 Hz, 4H; Ar-H), 6.52 (s, 2H; =CH₂), 5.89 (s, 2H; =CH₂), 4.14 (m, 8H; OOC-CH₂-, SO₂-CH₂-), 3.62 (m, 12H; -CH₂-O-CH₂-CH₂-), 2.43 (s, 6H; Ar-CH₃).
¹³C-NMR (50 MHz, CDCl₃, δ): 164.9 (C=O), 144.9 (C4), 135.4 (C4), 133.6 (C2), 129.7 (C3), 128.9 (C4), 128.8 (C4), 70.7 (C2), 68.8 (C2), 64.5 (C2), 57.5 (C2), 21.6 (C1).

### Beispiel 3:

### Synthese von Triethylenglykol-bis[2-(toluol-4-sulfonylmethyl)-acrylat] (3)

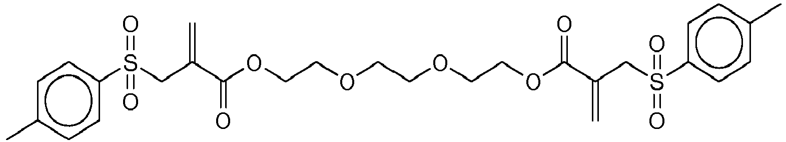

In einem Braunglaskolben wurden nach Beispiel 1 zur Herstellung von MBSI 1 Natrium-p-toluolsulfinat (39,20 g, 0,22 mol) mit Iod (55,83 g, 0,22 mol) umgesetzt und aufgearbeitet. Der gelbe Feststoff wurde in Dichlormethan (300 ml) gelöst. Triethylenglykoldimethacrylat (28,63 g, 0,10 mol) wurde zugegeben und das Reaktionsgemisch wurde bei RT gerührt. Nach 24 h wurde Triethylamin (22,26 g, 0,22 mol) zugetropft. Die rotbraune Lösung wurde 2 h bei Umgebungstemperatur gerührt und anschliessend am Rotationsverdampfer eingeengt. Das dunkelbraune Öl wurde in n-Hexan/Ethylacetat 1:1 (100 ml) aufgenommen und über eine mit Kieselgel gefüllte Fritte filtriert (Kieselgel 60, n-Hexan/Ethylacetat 1:1). Das Filtrat wurde am Rotationsverdampfer eingeengt. Der Rückstand wurde in Ethylacetat (400 ml) gelöst und mit Triethylamin (22,26 g, 0,22 mol) versetzt. Die bräunliche Lösung wurde 6 h am Rückfluss erhitzt. Nach dem Abkühlen wurde die Reaktionslösung mit Salzsäure (1N; 2x 200 ml) und Wasser (200 ml) gewaschen, über Na₂SO₄ getrocknet, filtriert und am Rotationsverdampfer eingeengt. Das bräunliche Öl wurde mittels Säulenchromatographie gereinigt (Kieselgel 60, n-Hexan/Ethylacetat 1:2; R_{f} = 0.35). Man erhielt 48.44 g (81% Ausbeute) eines gelben Öls.
¹H-NMR (200 MHz, CDCl₃, δ): 7.73 (d, *J =* 8.2 Hz, 4H; Ar-H), 7.32 (d, *J* = 8.2 Hz, 4H; Ar-H), 6.52 (s, 2H; =CH₂), 5.89 (s, 2H; =CH₂), 4.14 (m, 8H; OOC-CH₂-, SO₂-CH₂-), 3.62 (m, 12H; -CH₂-O-CH₂-CH₂-), 2.43 (s, 6H; Ar-CH₃);
¹³C-NMR (50 MHz, CDCl₃, δ): 164.9 (C=O), 144.9 (C4), 135.4 (C4), 133.6 (C2), 129.7 (C3), 128.9 (C4), 128.8 (C4), 70.7 (C2), 68.8 (C2), 64.5 (C2), 57.5 (C2), 21.6 (C1);

### Beispiel 4:

### Synthese von 2-Propensäure-2-[(diethoxyphosphinyl)-methyl]-ethylester (4)

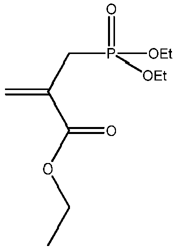

Für die Synthese von 4 wurde Ethyl-2-bromomethylacrylat (1,5g, 0,008mol) mit destillierten Triethylphosphit (1,3g, 0,008mol) 7h unter Ar-Atmosphäre im 10mL Rundkolben auf Rückfluss erhitzt und anschließend 12h bei RT umgesetzt. Das Produkt wurde in einen Birnenkolben überführt und EtBr (Sp=38°C bei 1,013 bar) bei 5 mbar entfernt. Anschließend wurde mittels MPLC mit Petrolether (PE)/Ethylacetat (EE) 3:2 gereinigt (Rf ∼ 0,13). Die Ausbeute an säulenchromatographisch gereinigtem 4 betrug 1,6g (82% der Theorie).
¹H-NMR (200 MHz, CDCl₃, δ): 0, 92-1, 34 (9,1 1 H, m, 3x -CH₂-CH₃), 2,77 (1 H, d, J=0,78 Hz, =C(COOEt)-CH₂-PO(OEt)₂), 2,87 (1 H, d, J=0,78 Hz, =C(COOEt)-CH₂-PO(OEt)₂), 3,87-4,18 (6,1 H, m,3x -O-CH₂-CH₃), 5,73 (1 H, dd, J=5,46 Hz, J=0,78 Hz, H₂C=C(COOEt)-), 6,22 (1 H, dd, J=5,66 Hz, J=0,58 Hz, H₂C=C(COOEt)-).

### Beispiel 5:

### Synthese von 2-Propensäure-2-[(dodecylthio)methyl]-ethylester (5)

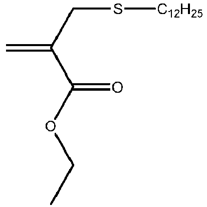

Für die Synthese von 5 wurden *Triethylamin* (1,1g, 0,011mol) und frisch destilliertes *Dodecylthiol* (1,6g, 0,008mol) mit 10mL THF in einem 50mL Rundhalskolben vorgelegt und *Ethyl-2-bromomethylacrylat* (1,5g, 0,008mol) mit 9mL THF in den Reaktionskolben gespült. Bei Zugabe des Acrylates fiel sofort weißer, feiner Niederschlag aus. Das Ende der Reaktion, nach 21h rühren bei Raumtemperatur (RT), wurde mittels Dünnschichtchromatographie (DC) bestätigt. Die Aufarbeitung erfolgte durch Lösen von nicht umgesetztem Salz in 15mL deion. Wasser. Die wässrige Phase wurde 3x mit 15 mL Petrolether extrahiert, die vereinten organischen Phasen über Na₂SO₄ getrocknet und PE am Rotationsverdampfer abgezogen. Anschließend wurde mittels Mitteldruck-Flüssigkeits-Chromatographie (MPLC) mit PE/EE 10:1 gereinigt (R_{f} ∼ 0,46 (PE/EE 12:1)). Die Ausbeute an säulenchromatographisch gereinigtem 5 betrug 1,2g (50% der Theorie).
¹H-NMR (200 MHz, CDCl₃, δ): 0,85 (3,5 H, t, J=6,46 Hz, -C₁₀H₂₀-CH₃), 1,10-1,65 (27,1 H, m, -CH₂-C₁₀H₂₀-CH₃ and -O-CH₂-CH₃), 2,42 (2,1 H, t, J=7,24 Hz, -S-CH₂-C₁₁H₂₃), 3,35 (2 H, s, =C(COOC₂H₅)-CH₂-S-), 4,21 (2,2 H, q, J=7,11 Hz, -O-CH₂-CH₃), 5,61 (1H, d, J=1,17 Hz, H₂C=C(COOC₂H₅)-), 6,17 (1 H, d, J=1,98 Hz, H₂C=C(COOC₂H₅)-).

### Beispiel 6:

### Synthese von 2-{[2-(Ethoxycarbonyl)-2-propenyl]-sulfanyl]-methyl}-acrylsäureethylester (6)

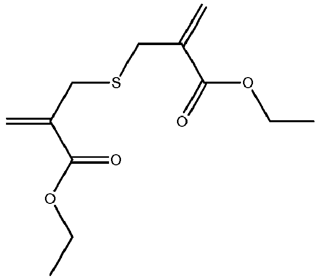

Für die Synthese von *6* wurde *Ethyl-2-bromomethylacrylat* (10,0g, 0,052mol) in einem 50mL Rundkolben vorgelegt und frisch aus deion. H₂O umkristallisiertes *Natriumsulfid* (5,9g, 0,021mol) in 2mL deion. H₂O auf einmal zugegeben und mittels 8mL H₂O nachgespült. Es wurde 24h bei RT gerührt und nach Kontrolle der Reaktion mittels DC mit 15mL deion. H₂O verdünnt, um entstandenes Salz zu lösen. Es wurde 3x mit 15mL Petrolether extrahiert, die vereinten organischen Phasen mit gesättigter Kochsalzlösung extrahiert und anschließend über Na₂SO₄ getrocknet. Nach Abziehen des Lösungsmittels am Rotationsverdampfer, erfolgte die Reinigung mittels MPLC in PE/EE 6:1 (R_{f} ∼ 0,49) und ergab 4,0g *6* (30% theoretische Ausbeute).
¹H-NMR (200 MHz, CDCl₃, δ): 1,18 (6,1 H, t, J=7,13 Hz, 2x -CH₂-CH₃), 3,20 (3,8 H, d, J=0,58 Hz, 2x =C(COOC₂H₅)-CH₂-S-), 4,10 (4,1 H, q, J=7,11 Hz, -O-CH₂-CH₃), 5,55 (2 H, d, J=0,98 Hz, 2x H₂C=C(COOC₂H₅)-), 6,09 (2 H, d, J=0, 98 Hz, 2x H₂C=C(COOC₂H₅)-).

### Beispiel 7:

### Synthese von 2-{[2-(Ethoxycarbonyl)-2-propenyl]-sulfonyl]-methyl}-acrylsäureethylester (7)

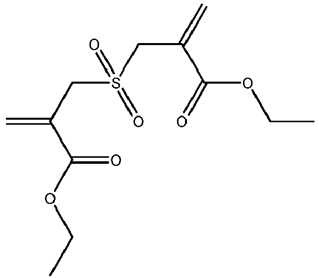

Für die Synthese von 7 wurde Verbindung 6 (3,0g, 0,012mol) in 100mL DMF in einem 500mL Rundhalskolben vorgelegt, *Kaliumperoxosulfat* (13,9g, 0,045mol) zugegeben und mit 200 mL DMF nachgespült. Es wurde 4h bei RT unter Ar-Atmosphäre gerührt und nach Kontrolle der Reaktion mittels DC deion. H₂O (900 mL) zugesetzt. Es wurde 3x mit 130mL Diethylether, 1x mit 150 mLg gesättigter Kochsalzlösung extrahiert und über Na₂SO₄ getrocknet. Nach Abziehen des Lösungsmittels am Rotationsverdampfer, erfolgte die Reinigung mittels MPLC in PE/EE 10:1 (R_{f} ∼ 0,67 (PE/EE 1:1)) und ergab 1,2g *7* (37% theoretische Ausbeute).
¹H-NMR (200 MHz, CDCl₃, δ): 1,24 (6,4 H, t, J=7, 14 Hz, 2x -CH₂-CH₃), 4,03 (4,1 H, s, 2x =C(COOC₂H₅)-CH₂-S-), 4,18 (4,1 H, q, J=7,17 Hz, -O-CH₂-CH₃), 6,08 (2 H, s, 2x H₂C=C(COOC₂H₅)-), 6,53 (2 H, s, 2x H₂C=C(COOC₂H₅)-).

### Beispiel 8:

### Synthese von 2-(Tosylmethyl)acrylonitril (8)

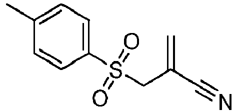

*Tosyliodid* (4,88 g, 17 mmol) und *Methacrylnitril* (1,16 g, 17 mmol) wurden in 100 ml *Tetrachlorkohlenstoff* gelöst und bei Raumtemperatur 4 h gerührt. Anschließend wurden das Lösungsmittel und entstandenes *Iod* im Vakuum abgedampft. Nach der erneuten Zugabe von 100 ml *Tetrachlorkohlenstoff* wurde die Lösung mit 4 Äquivalenten Triethylamin versetzt und 12 h auf Rückfluss erhitzt. Die resultierende braune Lösung wurde mit 5%iger *Natriumdithionitlösung* (2x 20 ml), 1N *HCl* (1x 20 ml) und gesättigter NaCl Lösung (1x 20 ml) gewaschen um restliches Iod und Triethylamin zu entfernen. Die gesammelten organischen Phasen wurden über 15 g Na₂SO₄ getrocknet und das Lösungsmittel am Rotationsverdampfer abgezogen. Das Rohprodukt wurde mit reinem Dichlormethan als Laufmittel säulenchromatographisch gereinigt. Die Ausbeute betrug 789 mg (21% d.Th.) 2-*(Tosylmethyl)acrylonitril* 8 als feine weiße Nadeln.
¹H NMR (200 MHz, CDCl3) δ= 7,73 (d, 8,45 Hz, 2 H; Ar-H), 7,33 (d, J= 8,45 Hz, 2 H; Ar-H), 6,15 (s, 1 H; C=H2), 5,94 (s, 1 H; C=H2), 3,84 (s, 2 H; -SO2-CH2-), 2,41 (s, 3 H; Ar-CH3) ppm.

### Beispiel 9:

### Synthese von 1-Methyl-4-((2-phenylallyl)sulfonyl)benzol (9)

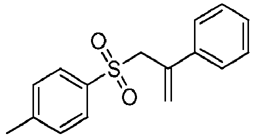

*Methylstyrol* (2,95 g, 25 mmol), *Tosylchlorid* (4,77 g, 25 mmol), *Cu(I)Cl* (2,48 g, 25 mmol) und *Triethylamin* (2,53 g, 25 mmol) wurden in 70 ml trockenem *Acetonitril* vorgelegt und 3 h unter Argonatmosphäre auf Rückfluss erhitzt. Nach dem Abziehen des Lösungsmittels am Rotationsverdampfer, wurde das Rohprodukt in 30 ml Dichlormethan aufgenommen und mit 1N HCl (2x 20 ml), gesättigter NaHCO₃ Lösung (1x 20 ml) und gesättigter NaCl Lösung (1x 20 ml) gewaschen. Nach säulenchromatigraphischer Reinigung mit Dichlormethan als Laufmittel wurde *1-Methyl-4-((2-phenylallyl)sulfonyl)benzol* 9 in einer Ausbeute von 3,60 g (53% d.Th.)erhalten.
¹H NMR (200 MHz, CDCl₃) δ= 7,59 (d, J= 7,64 Hz, 2 H; Ar-H), 7,5-6,9 (m, 7 H; Ar-H), 5,51 (s, 1 H; C=H₂), 5,14 (s, 1 H; C=H₂), 4,18 (s, 2 H; -SO₂-CH₂-), 2,32 (s, 3 H; Ar-CH₃) ppm.

### Beispiel 10:

### Synthese von 2-(Tosylmethyl)acrylsäure (10)

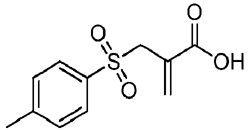

*Brommethacrylsäure* (8,25 g, 50 mmol) wurde in 250 ml heißem MeOH gelöst und mit NaOH (2 g, 50 mmol) versetzt. Anschließend wurde Toluylnatriumsulfinat (8,91 g, 50 mmol) portionsweise zugegeben und 2 h auf Rückfluss erhitzt. Nach dem Abziehen des Lösungsmittels an einem Rotationsverdampfer wurde der feste Rückstand in 500 ml Wasser aufgenommen und *2-(Tosylmethyl)-acrylsäure* 10 mit 1 N HCl ausgefällt. Ausbeute 7,44 g (62 %d.Th.).
¹H NMR (200 MHz, CDCl₃) δ= 8,81 (bs, 1H), 7,67 (d, J= 8,6 Hz, 2 H; Ar-H), 7,27 (d, J= 8,6 Hz, 2 H; Ar-H), 6,55 (s, 1 H; C=H₂), 5,94 (s, 1 H; C=H₂), 4,04 (s, 2 H; -SO₂-CH₂-), 2,36 (s, 3 H; Ar-CH₃) ppm.

### Beispiel 11:

### Synthese von N-Methyl-N-propyl-2-(tosylmethyl)acrylamid (11)

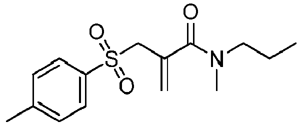

*2-(Tosylmethyl)acrylsäure* 10 (3,00 g, 12,5 mmol) wurde in 30 ml *Thionylchlorid* 2 h lang auf Rückfluss erhitzt. Nach dem Abziehen des überschüssigen SOCl₂ wurde das Säurechlorid in 100 ml Dichlormethan aufgenommen und bei 0 °C langsam mit 6 Äquivalenten *Propylmethylamin* versetzt. Nach 12 h Rühren bei RT wurde das Lösungsmittel am Rotationsverdampfer abgezogen und das Rohprodukt, nach der Aufnahme in 20 ml Dichlormethan, mit 1 N HCl (2 x 20 ml) und gesättigter NaCl Lösung (1 x 20 ml) gewaschen. Nach säulenchromatographischer Reinigung (PE:EE (1:1) + 0,5 %iger Essigsäure) wurden 701 mg (19 %d.Th.) N-*Methyl-N-propyl-2-(tosylmethyl)acrylamid* 11 erhalten.
¹H NMR (200 MHz, CDCl₃) δ= 7,78 (d, J= 8,1 Hz, 2 H; Ar-H), 7,34 (d, J= 8,1 Hz, 2 H; Ar-H), 5,51 (bs, 1 H; C=H₂), 5,41 (s, 1 H; C=H₂), 4,09 (s, 2 H; -SO₂-CH₂-), 3,5-2,7 (m, 5 H; N-CH₃, N-CH₂-CH₂-CH₃), 2,38 (s, 3 H; Ar-CH₃), 1,7-1,3 (m, 2H; N-CH₂-CH₂-CH₃), 0,85 (t, J= 7,5 Hz, 3 H; N-CH₂-CH₂-CH₃) ppm.

### Beispiel 12:

### Synthese von 14-Methyl-13-oxo-3,6,9,12-tetraoxapentadec-14-en-1-yl 2-(tosylmethyl)acrylat (12)

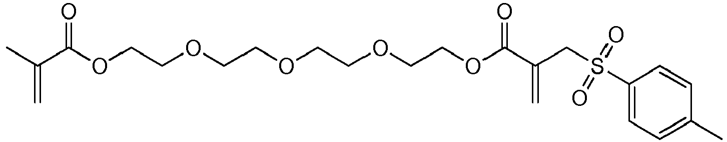

Tetraethylenglykoldimethacrylat (TTEGDMA, 14,6 g, 44,7 mmol) und 4-Toluolsulfonyliodid (12,6 g, 44,7 mmol) wurden gemeinsam in CH₂Cl₂ (ca. 100 ml) bei Raumtemperatur im Gelblicht gerührt. Die Synthese erfolgte anlog der Synthese von Beispiel 2. Es wurde Triethylamin (22,6 g, 223,4 mmol) zugetropft (Feststoff fiel aus). Das Rohprodukt wurde mit einem Gemisch aus PE/EE 1/3 säulenchromatographisch gereinigt. (R_{f} ∼ 0,38). Ausbeute 24%.
¹H-NMR (200 MHz, CDCl₃, δ): 7,70 (d, *J =* 8,2 Hz, 2H; Ar-H), 7,31 (d, *J* = 8,2 Hz, 2H; Ar-H), 6,49 (s, 1H; =CH₂), 6,10 (m, 1H; =CH₂), 5,86 (s, 1H; =CH₂), 5,50 (m, 1H; =CH₂), 4,27 (m, 2H; -OOC-CH₂-), 4,12 (m, 4H; -OOC-CH₂-, -SO₂-CH₂-), 3,71 (m, 2H; OOC-CH₂-CH₂-), 3, 63 (m, 10H; -CH₂-O-CH₂-CH₂-O-CH₂-CH₂-), 2,41 (s, 6H; Ar-CH₃) 1, 92 (m, 3H; -CO-C-CH₃).
¹³C-NMR (50 MHz, CDCl₃, δ): 164,9 (C=O), 145,0 (C4), 136,2 (C4) 135,5 (C4), 133,7 (C2), 129,8 (C3), 129,0 (C4), 128,9 (C3), 125,9 (C2), 70,7 (C2), 69,2 (C2), 68,9 (C2), 64,6 (C2), 64,0 (C2), 57,7 (C2), 21,8 (C1), 18,4 (C1).

### Beispiel 13:

### Synthese von 2-(Methylsulfonylmethyl)-acrylsäureethyl-ester (13)

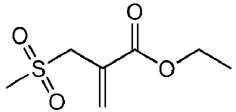

Ethyl-2-(bromomethyl)acrylat (1,1 g, 5,8 mmol), Natriummethansulfinat (0,7 g, 6,7 mmol) und 0,1 g Polyethylenoxid 400 wurden unter Argon-Atmosphäre in 10 mL absolutem THF vorgelegt. Anschließend wurde für 20 Stunden auf Rückfluss erhitzt, wobei der Reaktionsfortschritt mittels NMR und DC überwacht wurde. Nach vollständiger Reaktion wurde die Reaktionslösung mit 10 ml deionisiertem Wasser und 10 ml Diethylether verdünnt. Die wässrige Phase wurde dreimal mit je 25 ml Diethylether extrahiert. Die vereinigten organischen Phasen wurden danach mit gesättigter Kochsalzlösung gewaschen, über Natriumsulfat getrocknet und am Rotationsverdampfer eingedampft. Das erhaltene Rohprodukt wurde säulenchromatographisch mit einem Gemisch aus PE/EE 1/1 gereinigt. (R_{f} ∼ 0,45). Ausbeute 33%.
¹H-NMR (200 MHz, CDCl₃, δ): 6,64 (s, 1H; =CH₂), 6,15 (s, 1H; =CH₂), 4,28 (q, *J =* 7,1 Hz, 2H; -COO-CH₂-CH₃), 4,06 (s, 2H; -SO₂-CH₂-C-), 2,90 (s, 3H; -SO₂-CH₃), 1,33 (t, *J* = 7,1 Hz, 3H; -COO-CH₂-CH₃).
¹³C-NMR (50 MHz, CDCl₃, δ): 165,3 (C=O), 133,9 (C2), 129,1 (C4), 61,9 (C2), 56,4 (C2), 40,5 (C1), 14,1 (C1).

### Beispiel 14:

### Synthese von 2-Methylen-3-[(4-methylphenyl)sulfonyl]butter-säuremethyl ester (14)

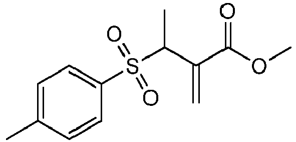

(Z)-Methyl-2-(bromomethyl)but-2-enoat (2,7 g, 14,0 mmol) und 0,5 g Polyethylenoxid 400 wurden in 15 ml absolutem THF vorgelegt und auf -20 °C abgekühlt. Anschließend wurde Natrium-p-toluolsulfinat (0,8 g, 4,7 mmol) langsam zugegeben. Bei -20 °C wurde für 10 Stunden gerührt. Die Aufreinigung erfolgte säulenchromatographisch mit einem Gemisch aus PE/EE 2/1. (R_{f} ~ 0,50). Ausbeute 36%.
¹H-NMR (200 MHz, CDCl₃, δ): 7,69 (d, *J =* 8,2 Hz, 2H; Ar-H), 7,30 (d, *J* = 8,2 Hz, 2H; Ar-H), 6,53 (s, 1H; =CH₂), 5,98 (s, 1H; =CH₂), 4,58 (q, *J* = 7,2 Hz, 1H; -SO₂-CH-), 3,60 (s, 3H; -COO-CH₃), 2,42 (s, 3H; Ar-CH₃), 1,54 (d, *J =* 7,2 Hz, 3H; -SO₂-CH-CH₃).

### Beispiel 15:

### Synthese des thermolabilen Dimethacrylats 3-Oxo-2-(2,2,4-trimethyl-6-{2-[2-(methacryloyloxy)-ethoxycarbonyl]-3-oxo-butyrylamino}-hexylcarbamoyl)-buttersäure-2-(methacryloyloxy)-ethylester

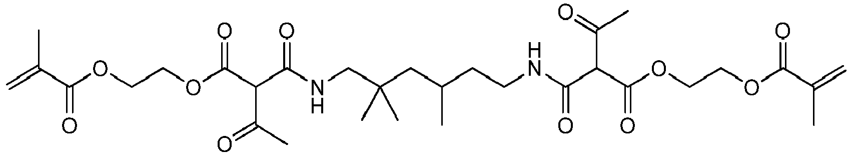

In einer unter Argon ausgeheizten Apparatur wurde Natrium (0,50 g; 2,2 mmol) in Diethylether (400 ml) vorgelegt und (2-Acetoacetoxy)ethylmethacrylat (85,69 g; 0,40 mol) wurde zugetropft. Das Reaktionsgemisch wurde 20 h bei RT gerührt und anschließend im Eisbad auf 0 °C abgekühlt. 2,2,4-Trimethyl-1,6-diisocyanatohexan (42,06 g; (0,20 mol) wurde langsam zugetropft. Das Reaktionsgemisch wurde nach beendeter Zugabe noch 2 h unter Eiskühlung und anschließend bei Umgebungstemperatur weitergerührt. Nach 48 h wurde die Suspension filtriert, und das Filtrat wurde mit Salzsäure (1*N*; 2x 100 ml), Wasser (100 ml) und gesättigter wässriger NaCl-Lösung (100 ml) gewaschen, über Na₂SO₄ getrocknet, filtriert und am Rotationsverdampfer eingeengt. Das gelbliche Öl wurde in Dichlormethan (100 ml) gelöst und über eine mit Kieselgel gefüllte Fritte filtriert (Kieselgel 60, *n*-Hexan/Ethylacetat 1:1). Das Filtrat wurde am Rotationsverdampfer eingeengt und der Rückstand wurde am Feinvakuum getrocknet. Man erhielt das Dimethacrylat 4 (105,42 g, 83% Ausbeute) als gelbliches Öl.
¹H-NMR (CDCl₃, 400 MHz): δ = 0.95 (s, 6H), 0. 98 - 1.02 (m, 3H), 1.09 - 1.68 (m, 5H), 1.95 (s, 6H), 2.41 - 2.43 (m, 6H), 3.05-3.36 (m, 4H), 4.44 (s, 8H), 5.61 (s, 2H), 6.13 (s, 2H), 9.10-9.30 (m, 2H), 18.70 - 18.73 (m, 2H).

### Beispiel 16:

### Herstellung von Kompositen mit dem Übertragungsreagenz 1 aus Beispiel 1

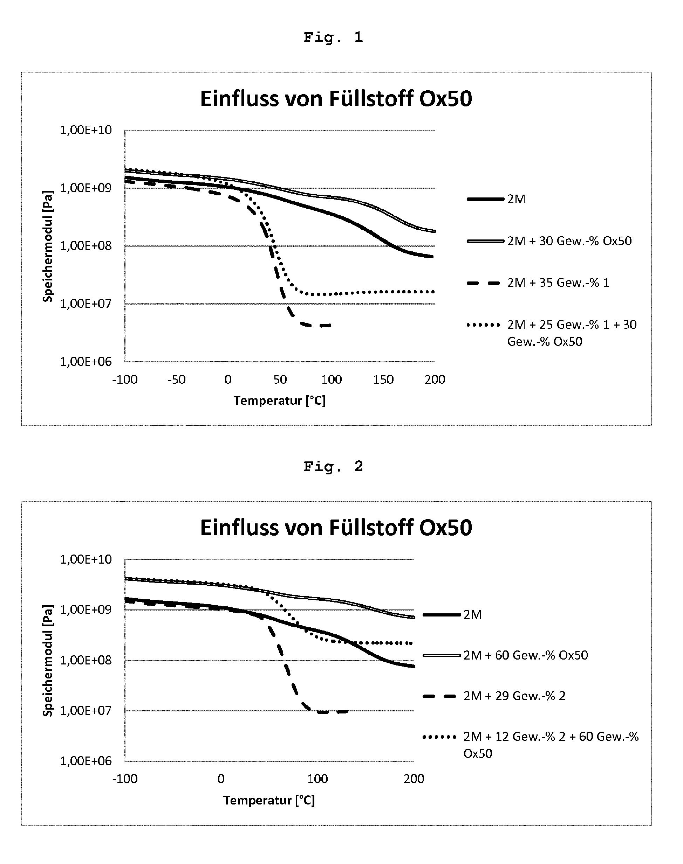

Es wurde eine 1/1-Mischung (mol/mol) der Monomere UDMA und D₃MA hergestellt (Harzmischung 2M). Ein Teil dieser Mischung wurde mit dem Monomer 1 aus Beispiel 1 gemischt. Die zweite Mischung wies die folgende Zusammensetzung auf: UDMA (39 Gew.-%), D₃MA (26 Gew.-%) und 1 (35 Gew.-%). Beide Mischungen wurden mit dem Photoinitiator Ivocerin® (1 Gew.-%) versetzt. Kompositpasten auf der Basis dieser Mischungen wurden durch Zugabe von 30 Gew.-% der pyrogenen Kieselsäure Ox50 hergestellt. Die Kompositpaste mit dem Monomer 1 hatte die Gesamtzusammensetzung: UDMA (27 Gew.-%), D₃MA (18 Gew.-%), 1 (24,3 Gew.-%), Initiator (0,7 %) und Ox50 (30 Gew.-%). Die Formulierungen wurden in Silikonformen gegossen und in einem Lumamat 100 (Ivoclar AG) mit dem Programm 2 (P2: 10 min Bestrahlung mit einer Intensität von ca. 20 mW/cm²) polymerisiert. Die Stäbe wurden gewendet und abermals mit P2 ausgehärtet. Die Probestäbchen wurden geschliffen und dann auf einem AntonPaar Rheometer MCR301 mit einem CTD-Ofen (Convection Temperature Control) und einer eingesetzten Festkörpereinspannvorrichtung (SRF12 für rechteckige Querschnitte bis 12 mm) vermessen. Die eingestellte Heizrate betrug 2 °C/min. Alle Proben wurden von -100 °C auf 200 °C aufgeheizt und mit einer konstanten Frequenz von 1 Hz und 0,1% Auslenkung oszilliert. Die in Fig. 1 dargestellten Speichermodulkurven zeigen, dass die Zugabe des Übertragungsreagenz 1 sowohl bei der ausgehärteten Harzprobe als auch im Falle des Komposits gleichermassen zu einer Verringerung der Glasübergangstemperatur und zu einem tieferen und deutlich schmaleren Glasübergangsbereich führt.

| **Formulierung** | **T_{G} [°C]** |
|---|---|
| 2M^{a)} * | 148 |
| 2M + Ox50* | 162 |
| 2M + Monomer 1 | 50 |
| 2M + Monomer 1 + Ox50 | 50 |

| | |
|---|---|
| ^{a)} 2M: UDMA/D₃MA (1/1) * Vergleichsbeispiel | |

### Beispiel 17:

### Herstellung von Kompositen mit dem Übertragungsreagenz 2 Beispiel 2

Analog zu Beispiel 4 wurden Prüfkörper hergestellt und untersucht. Dabei wurden folgende Formulierungen verwendet: 1/1-Mischung (mol/mol) von UDMA und D₃MA (Mischung 2M) und eine Mischung von UDMA (43 Gew.-%), D₃MA (28 Gew.-%) mit Monomer 2 (29 Gew.-%). Entsprechende Kompositpasten wurden durch Zugabe von 60 Gew.-% Ox-50 erhalten. Die Kompositpaste mit dem Monomer 2 hatte die folgende Gesamtzusammensetzung: UDMA (17 Gew.-%), D₃MA (11 Ges.-%), 2 (12 Gew.-%) und Ox50 (60 Gew.-%). Fig. 2 zeigt wiederum den Speichermodul in Abhängigkeit von der Temperatur für die ungefüllten und gefüllten Harze. Die in Fig. 2 gezeigten Speichermodulkurven zeigen, dass die Zugabe des Übertragungsreagenz 2 (Beispiel 2) sowohl bei dem ungefüllten Harz als auch im Falle des Komposits zu einem deutlichen schmaleren Glasübergangsbereich mit verringerter Glasübergangstemperatur (T_{G}) führt.

| **Formulierung** | **T_{g} [°C]** |
|---|---|
| 2M^{a)} * | 148 |
| 2M + Ox50* | 157 |
| 2M + Monomer 2 | 73 |
| 2M + monomer 2 + Ox50 | 78 |

| | |
|---|---|
| ^{a)} 2M: UDMA/D₃MA (1/1) * Vergleichsbeispiel | |

### Beispiel 18:

### Bestimmung der Debonding-on-Demand-Eigenschaften

Ein zweikomponentiger, licht- und dualhärtendes Befestigungszement Variolink II (Ivoclar Vivadent AG, der Zement basiert auf einer Monomermischung aus Bis-GMA, UDMA und TEGDMA, als Füllstoffe sind Bariumglas, Ba-Al-Fluorosilikatglas, SiO₂-ZrO₂-Mischoxid und Ytterbiumtrifluorid enthalten) wurde mittels Monomer 3 aus Beispiel 3 für Kronenabzugversuche modifiziert. Hierzu wurden 9,066 g der Komponente *Variolink II Base transparent* mit 0,934 g Monomer 3 für 2x3 Minuten auf höchster Stufe bei 0,2 bar Restdruck gemischt (Planeten-Mischer DAC 600.2 VAC-P, Fa. Hauschild & Co KG, Hamm, Deutschland). Auf dieselbe Weise wurden 9,017 g der Komponente *Variolink II Katalysator transparent*/*dünn* mit 0,983 g Monomer 3 modifiziert.

Die Untersuchung der Kronenabzugkräfte wurde sowohl mit dem modifizierten Material Variolink II (s.o.) als auch mit unmodifiziertem Variolink II durchgeführt. Hierzu wurden Testkronen auf dazu passende Stümpfe aus Zirkoniumdioxid (Kegelstumpfgeometrie: Kegelwinkel 40°, Höhe 3,3 mm, Mantelfläche 56,3 mm², vorgegebener Zementspalt 50 µm) befestigt. Die Verbundflächen des Testsystems wurden zuvor jeweils frisch sandgestrahlt (Al₂O₃ 110 µm, 1 bar) und mit dem Haftvermittler Monobond Plus (Ivoclar Vivadent AG, Einwirkzeit 60s) oberflächenkonditioniert. Für die Befestigung wurden jeweils 250 mg Base- und Katalysator-Paste (Testgruppen 1 und 2: *Variolink II Base transparent, Variolink II Katalysator transparent*/*dünn;* Testgruppen 3 und 4: gemäss obenstehendem Beispiel modifizierte *Variolink II Base transparent,* gemäss obenstehendem Beispiel modifizierter *Variolink II Katalysator transparent*/*dünn*) von Hand mittels eines Spatels auf einem Mischblock innerhalb von 10 s vermischt und etwa die Hälfte der Mischung in die Krone gefüllt. Daraufhin wurde die Krone auf den Stumpf gesetzt und mit einem statischen Gewicht von 2 kg belastet. Direkt im Anschluss wurden die Überschüsse mittels Microbrush (Microbrush, Grafton, USA) entfernt und dann, ca. 30 s nach Beginn der Belastung, mittels eines Lichtpolymerisationsgerätes (Bluephase G2, Ivoclar Vivadent AG; High Power) von 2 Seiten für jeweils 40 s belichtet. Danach wurde der Testkörper entlastet und bis zur Messung für mindestens 16 h bei 37 °C im Trockenschrank gelagert.

Für die Bestimmung der Kronenabzugkräfte wurden die Testkörper der Testgruppen 1 und 3 in einem auf 23 °C, die Testkörper der Gruppen 2 und 4 in einem auf 60 °C temperierten Wasserbad (Temperierbehälter für medizintechnische Versuche, Zwick-Röll GmbH & Co KG, Ulm, Deutschland) mittels einer Materialprüfmaschine (Z010, Zwick-Röll) vermessen. Vor Beginn der Messung wurden die Kronen jeweils für mindestens 2 Min. im entsprechenden Wasserbad temperiert. Anschliessend wurden die Kronen lagegeregelt bei einer Traversengeschwindigkeit von 1 mm/min. abgezogen, wobei die dabei auftretende höchste Kraft als Kronenabzugkraft registriert wurde. Die Ergebnisse belegen eine deutlich reduzierte Kronenabzugskraft bei 60 °C des Zementes mit der erfindungsgemäßen Verbindung 3.

### Ergebnis (Kronenabzugkräfte)

| **Testgruppe 1*** | **Testgruppe 2*** | **Testgruppe 3** | **Testgruppe 4** |
|---|---|---|---|
| 1101 N | 808 N | 1259 N | 558 N |
| +/- 320 N | +/- 168 N | +/- 238 N | +/- 264 N |

| | | | |
|---|---|---|---|
| * Vergleichsbeispiel | | | |

### Beispiel 19:

### Herstellung und Charakterisierung von Polymeren mit Dimethacrylaten und Übertragungsreagenzien

Es wurde eine 1/1-Mischung (mol/mol) von UDMA und D₃MA (2M) sowie Mischungen von UDMA, D₃MA und jeweils einem Übertragungsreagenz (Verbindungen Nr. 1, 2, 5-14) gemäß Tabelle 3 hergestellt. Die Formulierungen enthielten zusätzlich 1 Gew.-% Ge-Initiator (Ivocerin). Zur Überprüfung der Photoreaktivität wurden die hergestellten Formulierungen mit einem Photorheometer (Modell MCR 302 WESP, Anton Paar) vermessen. Es wurde ein Platte/Platte- Messsystem des Typs PP25 verwendet, und der Messspalt wurde auf 0,1 mm eingestellt. Vor und während der Aushärtung mit einer UV-Lampe (Modell Omniucure 2000; 400-500 nm; 1 W/cm² bzw. 3 W/cm²) wurden Speicher- und Verlustmodul der Probe im Osziallationsmodus (1 % Auslenkung, 1 Hz) gemessen.

Als Maß für die auftretende Polymerisationsschrumpfung dient der erreichte Doppelbindungsumsatz (DBC) am Gelpunkt (Schnittpunkt von Speicher- und Verlustmodul). Der Doppelbindungsumsatz bis zum Gelpunkt führt nicht zum Aufbau von Spannungen, da die auftretende Polymerisationsschrumpfung durch Fliessprozesse kompensiert wird. Je höher der Doppelbildungsumsatz bis zum Gelpunkte ist, umso geringer sind folglich der Doppelbindungsumsatz und der Polymerisationsschrumpf im Gelzustand, was somit auch zu einer geringeren Polymerisationsschrumpfkraft führt. Zur Ermittlung des Glasüberganges wurden die Formulierungen in Silikonformen gegossen und in einem Lichtofen (Modell Lumamat 100, Ivoclar AG) mit dem Programm 2 (P2: 10 min Bestrahlung mit einer Intensität von ca. 20 mW/cm²) polymerisiert. Die Stäbe wurden gewendet und abermals mit P2 ausgehärtet. Die Probestäbchen wurden geschliffen und dann auf einem Rheometer (Modell MCR 302) mit einem CTD-Ofen (Convection Temperature Control) und einer eingesetzten Festkörpereinspannvorrichtung (SRF12 für rechteckige Querschnitte bis 12 mm) vermessen. Die eingestellte Heizrate betrug 2 °C/min. Alle Proben wurden von -100 °C auf 200 °C aufgeheizt und mit einer konstanten Frequenz von 1 Hz und 0,1 % Auslenkung oszilliert.

Die in Tabelle 3 gezeigten Glasübergangstemperaturen (Maxima der Verlustmodulkurven) zeigen, dass die Zugabe der Übertragungsreagenzien zu einem tieferen und deutlich schmaleren Glasübergangsbereich führt, was bei den erfindungsgemäßen Zusammensetzungen ein Debonding-on-Demand erheblich erleichtert. Außerdem ist ersichtlich, dass der Doppelbindungsumsatz am Gelpunkt durch die Übertragungsreagenzien erhöht wird. Damit ist eine geringere Schrumpfungsspannung zu erwarten, weil bis zum Gelpunkt Spannungen durch Fliessprozesse abgebaut werden können.

**Tabelle 3**

| **Formulierung** | **T_{G} [°C]** | **HWB [°C]** | **DBC [%]** |
|---|---|---|---|
| 2M^{a)} * | 150 | 152 | 18 |
| 2M^{c)} * | 148 | 145 | 18 |
| 2M + 35 Gew.-% 1 | 50 | 28 | b) |
| 2M + 29 Gew.-% 2 | 75 | 32 | 23 |
| 2M + 29 Gew.-% 5 | 48 | 51 | 30 |
| 2M + 14 Gew.-% 6 | 134 | 51 | 24 |
| 2M + 16 Gew.-% 7 | 68 | 51 | 29 |
| 2M + 6 Gew.-% 8^{c)} | 129 | 93 | 51 |
| 2M + 7 Gew.-% 9^{c)} | 126 | 82 | 28 |
| 2M + 6 Gew.-% 10^{c)} | 114 | 55 | 25 |
| 2M + 34 Gew.-% 11^{c)} | 74 | 57 | 36 |
| 2M + 45 Gew.-% 12 | 64 | 23 | b) |
| 2M + 20 Gew.-% 13 | 66 | 26 | b) |
| 2M + 26 Gew.-% 14 | 65 | 74 | b) |

| | | | |
|---|---|---|---|
| * Vergleichsbeispiel T_{G} Glasübergangstemperatur HWB Halbwertsbreite DBC Doppelbindungsumsatz am Gelpunkt ^{a)} 2M: UDMA/D₃MA (1/1) ^{b)} nicht gemessen ^{c)} Aushärtung mit 3 W/cm² | | | |

## Patentansprüche

1. Radikalisch polymerisierbarer Dentalwerkstoff, der mindestens eine Verbindung der Formel I: 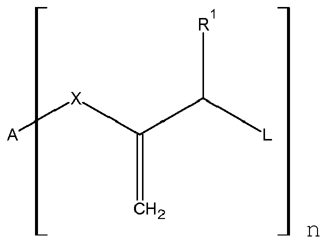 mit:
A = H; -CN; ein Phenylrest, der einen oder mehrere Substituenten, wie CH₃, C₂H₅, OH, OCH₃, O-COCH₃, eine polymerisationsfähige Vinyl-, (Meth)acryloyloxy- oder (Meth)acrylamidgruppe tragen kann; oder ein aliphatischer linearer oder verzweigter C₀-C₂₀-Alkylen-Rest, der durch ein oder mehrere 1,4-Phenylengruppen, Urethangruppen, O oder S unterbrochen sein kann und der endständig eine polymerisationsfähige Vinyl-, (Meth)acryloyloxy- oder (Meth)acrylamidgruppe tragen kann;
R¹ = H, ein aliphatischer linearer oder verzweigter C₁-C₉-Alkyl-Rest, Tolyl oder Phenyl;
L = SR², CO-Phenyl, SO₂R³, PO(R⁴R⁵), PO(OR⁶)(R⁷), PO(OR⁸)(OR⁹) oder Halogen, wobei
R²⁻⁹ jeweils unabhängig voneinander ein Phenylrest, der einen oder mehrere Substituenten, wie CH₃, C₂H₅, OH, OCH₃, -O-COCH₃, eine polymerisationsfähige Vinyl-, (Meth)acryloyloxy-, (Meth)acrylamidgruppe, -C(=CH₂)-COOR¹¹ oder -C (=CH₂) -CO-NR¹²R¹³ tragen kann; oder ein aliphatischer linearer oder verzweigter C₁-C₂₀-Alkylen-Rest sind, der durch O oder S unterbrochen sein kann und der endständig eine polymerisationsfähige Vinyl-, (Meth)acryloyloxy-, (Meth)-acrylamidgruppe, -C (=CH₂) -COOR¹¹ oder -C(=CH₂)-CO-NR¹²R¹³ tragen kann, wobei R¹¹⁻¹³ unabhängig voneinander jeweils ein linearer oder verzweigter C₁₋₆-Rest sind;
X = -COO-, -CON(R¹⁰)- oder entfällt, wobei die Bindung an A über O bzw. N erfolgt und
R¹⁰ = H; oder ein aliphatischer linearer oder verzweigter C₁-C₂₀-Alkylen-Rest ist, der durch ein oder mehrere O oder S unterbrochen sein kann und der endständig eine polymerisationsfähige Vinyl-, (Meth)acryloyloxy-, (Meth) acrylamidgruppe, -C(=CH₂)-COOR¹¹ oder -C(=CH₂)-CO-NR¹²R¹³ tragen kann, wobei R¹¹⁻¹³ unabhängig voneinander jeweils ein linearer oder verzweigter C₁₋₆-Rest sind;
n = eine ganze Zahl von 1 bis 6,
und mindestens eine thermolabile radikalisch polymerisierbaren Verbindung und/oder mindestens eine photolabile radikalisch polymerisierbare Verbindung enthält.

2. Dentalwerkstoff nach Anspruch 1, wobei die Variablen der Formel I die folgenden Bedeutungen haben:
A H, -CN, ein Phenylrest, ein aliphatischer linearer oder verzweigter C₁-C₁₅-Alkyl-Rest, der durch eine oder mehrere 1,4-Phenylengruppen, Urethangruppen oder O unterbrochen sein kann und der endständig eine polymerisationsfähige (Meth)acryloyloxygruppe tragen kann;
R¹ H, Phenyl, Tolyl, ein aliphatischer linearer C₁-C₃-Alkyl-Rest;
L SR² oder SO₂R³, wobei
R²⁻³ unabhängig voneinander jeweils ein aliphatischer linearer oder verzweigter C₁-C₂₀-Alkylen-Rest, der durch O unterbrochen sein kann und der endständig eine polymerisationsfähige (Meth)acryloyloxygruppe oder -C(=CH₂)-COOR¹¹ tragen kann, wobei R¹¹ ein linearer oder verzweigter C₁₋₃-Rest ist; oder ein Phenylrest, der einen oder mehrere Substituenten, vorzugsweise CH₃, C₂H₅, OCH₃ und/oder O-COCH₃ tragen kann;
X -COO- oder -CON(R¹⁰)-, wobei R¹⁰ Methyl ist, oder entfällt;
n 1 oder 2.

3. Dentalwerkstoff nach Anspruch 2, wobei die Variablen der Formel I die folgenden Bedeutungen haben:
A ein gesättigter, linearer aliphatischer Kohlenwasserstoffrest mit 1 bis 12 Kohlenstoffatomen, der durch eine oder mehrere 1,4-Phenylengruppen, Urethangruppen oder O unterbrochen sein und der eine Methacryloyloxygruppe tragen kann,
X -COO- oder -CON(R¹⁰)-, wobei R¹⁰ Methyl ist, oder entfällt;
R¹ H,
L -SO₂R³, wobei R³ CH₃ oder Tolyl ist,
n 1 oder 2.

4. Dentalwerkstoff nach Anspruch 3, wobei die Variablen der Formel I die folgenden Bedeutungen haben:
A ein gesättigter, linearer aliphatischer Kohlenwasserstoffrest mit 6 bis 12 Kohlenstoffatomen, der durch 1 bis 3 O-Atome unterbrochen sein kann,
X -COO-;
R¹ H,
L SO₂R³, wobei R³ CH₃ oder Tolyl ist,
n 1 oder 2.

5. Dentalwerkstoff nach einem der Ansprüche 1 bis 4, der als thermolabile radikalisch polymerisierbare Verbindung ein thermolabiles multifunktionelles (Meth)acrylat oder (Meth)acrylamid und/oder als photolabile radikalisch polymerisierbare Verbindung mindestens ein photolabiles multifunktionelles (Meth)acrylat oder (Meth)acrylamid enthält.

6. Dentalwerkstoff nach einem der Ansprüche 1 bis 5, der als thermolabile radikalisch polymerisierbare Verbindung ein Monomer enthält, das ausgewählt ist aus polyfunktionellen (Meth)acrylaten oder (Meth)acrylamiden mit mindestens einer thermolabilen Gruppe zwischen zwei (Meth)acryl- oder (Meth)acrylamidgruppen, dem Diels-Alder-Addukt aus Furfurylmethacrylat und N-(3-(Methacryloyloxy)propyl)-maleinimid, den Umsetzungsprodukten von N-Hydroxy-(meth)acrylamid mit Di- oder Triisocyanaten, Hexamethylen-1,6-diisocyanat (HDI), 2,2,4- Trimethylhexamethylen-1,6-diisocyanat oder dem HDI-Trimer, Produkten, die durch stöchiometrische Umsetzung von Di- oder Triisocyanaten mit 1-Hydroxymethylacrylsäureestern, 1-Hydroxymethylacrylsäureethylester, mit β-Ketoester(meth)acrylaten oder 2-Aceto-acetoxyethylmethacrylat erhältlich sind, thermolabilen Vernetzermonomeren, die Gas freisetzen, den Veresterungsprodukten von Azobis(4-cyanovaleriansäure) mit Hydroxyalkyl(meth)acrylaten, Hydroxyethyl(meth)acrylat, Hydroxypropyl(meth)acrylat, N-(Hydroxyalkyl)(meth)acrylamiden, N-(5-Hydroxypentyl)methacrylamid oder N-Methyl-N-(2-hydroxyethyl)acrylamid.

7. Dentalwerkstoff nach einem der Ansprüche 1 bis 6, der als photolabile radikalisch polymerisierbare Verbindung ein Monomer mit einer photolabilen Gruppe enthält, die ausgewählt ist aus Benzoinethern, Oxyalkylphenylacetophenonen, Dialkyloxyacetophenonen, Benzoyldiphenylphosphinoxiden, Dibezoylphenylphosphinoxiden, Dialkylbenzoyl- und Dialkyldibenzoylgermanium-Derivaten.

8. Dentalwerkstoff nach einem der Ansprüche 1 bis 7, der als photolabile radikalisch polymerisierbare Verbindung ein Monomer mit einer photolabilen Gruppe und zwei polymerisationsfähigen (Meth)acrylatgruppen, Bis-(4-methocryloyloxy-benzoyl-diethylgermanium, Bis-{4-[2-(methacryloyloxy)-di-ethylcarbamoyloxybenzoyl}diethylgermanium, Bis-[3-(methacryloyloxymethyl)-2,4,6-trimethylbenzoyl]phenylphosphinoxid oder Methacrylsäure-2-[2-(4-{2-methyl-2-[2-(methacryloyloxy)-ethylcarbamoyloxy]-propionyl}-phenoxy)-ethoxycarbonylamino]-ethylester enthält.

9. Dentalwerkstoff nach einem der Ansprüche 1 bis 8, der zusätzlich mindestens ein multifunktionelles (Meth)acrylat oder eine Mischungen von mono- und multifunktionellen (Meth)acrylaten enthält.

10. Dentalwerkstoff nach einem der Ansprüche 1 bis 9, der mindestens ein radikalisch polymerisierbares, säuregruppenhaltiges Monomer enthält.

11. Dentalwerkstoff nach einem der Ansprüche 1 bis 10, der zusätzlich mindestens einen Initiator für die radikalische Polymerisation, vorzugsweise einen Photoinitiator enthält.

12. Dentalwerkstoff nach einem der Ansprüche 1 bis 10, der
0,5 bis 60 Gew.-%, bevorzugt 1,0 bis 50 Gew.-% und besonders bevorzugt 1,0 bis 40 Gew.-% mindestens einer Verbindung der allgemeinen Formel I,
0,01 bis 5,0 Gew.-%, bevorzugt 0,1 bis 5,0 Gew.-% und besonders bevorzugt 0,1 bis 3,0 Gew.-% Initiator(en) für die radikalische Polymerisation, und
1 bis 60 Gew.-%, bevorzugt 5 bis 50 Gew.-% und besonders bevorzugt 5 bis 40 Gew.-% difunktionelle thermo- und/oder photolabile (Meth)acrylat(e) enthält, jeweils bezogen auf die Gesamtmasse des Dentalwerkstoffs.

13. Dentalwerkstoff nach Anspruch 12, der die folgende Zusammensetzung aufweist:
(a) 1 bis 80 Gew.-%, bevorzugt 10 bis 70 Gew.-% und besonders bevorzugt 10 bis 60 Gew.-% multifunktionelle (Meth)acrylat(e),
(b) 0,01 bis 5,0 Gew.-%, bevorzugt 0,1 bis 5,0 Gew.-% und besonders bevorzugt 0,1 bis 3,0 Gew.-% Initiator(en),
(c) 0,5 bis 60 Gew.-%, bevorzugt 1,0 bis 50 Gew.-% und besonders bevorzugt 1,0 bis 40 Gew.-% mindestens einer Verbindung der allgemeinen Formel I,
(d) 0 bis 50 Gew.-%, bevorzugt 0 bis 40 Gew.-% und besonders bevorzugt 0 bis 30 Gew.-% monofunktionelle (Meth)acrylat(e),
(e) 1 bis 60 Gew.-%, bevorzugt 5 bis 50 Gew.-% und besonders bevorzugt 5 bis 40 Gew.-% difunktionelle thermo- und/oder photolabile (Meth)acrylat(e),
(f) 0 bis 80 Gew.-%, besonders bevorzugt 0 bis 70 Gew.-% Füllstoff(e),
(g) 0 bis 40 Gew.-%, bevorzugt 0 bis 30 Gew.-% und besonders bevorzugt 5 bis 30 Gew.-% expandierende Additiv(e) und/oder Strahlungs-Wärmeumwandler,
(h) 0 bis 60 Gew.-%, bevorzugt 0 bis 50 Gew.-%, besonders bevorzugt 1 bis 50 Gew.-% Lösungsmittel und
(i) 0 bis 5 Gew.-%, bevorzugt 0 bis 3 Gew.-%, besonders bevorzugt 0,5 bis 3 Gew.-% sonstige Additiv(e),
jeweils bezogen auf die Gesamtmasse des Dentalwerkstoffs.

14. Dentalwerkstoff nach einem der Ansprüche 1 bis 13 zur intraoralen Verwendung als Adhäsiv oder Zement.

15. Verwendung eines Dentalwerkstoffs nach einem der Ansprüche 1 bis 13 zur extraoralen Herstellung oder Reparatur von Dentalrestaurationen.

16. Verwendung einer Verbindung der Formel I, in der die Variablen wie in einem der Ansprüche 1 bis 4 definiert sind, zur Herstellung eines Adhäsivs oder Zements mit Debonding-on-Demand-Eigenschaften.
